# EUROPEAN PATENT APPLICATION

(11) **EP 4 470 471 A1**
(43) Date of publication of application: **04.12.2024**
(21) Application number: 24722098.1
(22) Date of filing: 11.04.2024
(51) Int. Cl.: A61B 6/00

(54) **DIGITAL X-RAY IMAGING METHOD AND DIGITAL X-RAY IMAGING DEVICE**

(30) Priority: 11.04.2023 CN 202310426224
(71) Applicant: Shenzhen Mindray Bio-Medical Electronics Co., Ltd., Shenzhen, Guangdong 518057 (CN)
(72) Inventor: ZHANG, Jiye, Shenzhen Guangdong 518057 (CN); ZHANG, Zanchao, Shenzhen Guangdong 518057 (CN); LIU, Yanpeng, Shenzhen Guangdong 518057 (CN)
(74) Representative: KIPA AB
(86) International application number: PCT/CN2024/087332
(87) International publication number: WO 2024/213068

(57) **Abstract**

Disclosed are a digital X-ray imaging method and a digital X-ray imaging apparatus (01), comprising: when a to-be-exposed body part is a stitching body part, determining an exposure parameter set of the stitching body part, said exposure parameter set comprising a plurality of travel points that the X-ray source (10) passes along at least two directions and exposure parameters of the the X-ray source (10) at the multiple travel points, said at least two directions being capable of determining a two-dimensional surface or a three-dimensional space, then based on the exposure parameter set, controlling the X-ray source (10) to pass the multiple travel points along the at least two directions and emit X-rays to the stitching body part at each of the travel points passed according to exposure parameters corresponding thereto and controlling the detector (20) to receive X-rays penetrating the stitching body part to obtain a plurality of digital X-ray images, and stitching the plurality of digital X-ray images to obtain a radiograph of the stitching body part. Accordingly, exposure and stitching of the stitching body part can be implemented through the present disclosure.

## Description

### TECHNICAL FIELD

The present disclosure relates to X-ray imaging, in particular to digital X-ray imaging methods and digital X-ray imaging apparatus.

### BACKGROUND OF THE INVENTION

An X-ray imaging apparatus, such as a digital flat panel X-ray imaging apparatus, is a device that can reproduce X-ray radiographic images by processing X-rays that have been penetrated a human body and then collected by an X-ray detector (such as a flat panel detector).

The flat panel detectors available are standardized product with certain specifications, such as size of 43cm*43cm, which may cause inconvenience in practical use. Typically, for example, it is not possible to perform large-scale X-ray imaging.

### SUMMARY OF THE INVENTION

Considering the above problem, digital X-ray imaging methods and digital X-ray imaging apparatus are provided by the present disclosure, as detailed below.

In accordance with a first aspect, an digital X-ray imaging method provided in some embodiments may include:
displaying options of an exposable body part of an object under examination on a display interface, the options of exposable body part comprising at least an option of a stitching body part;
in response to a selection instruction on the options of the exposable body part, determining a to-be-exposed body part of the object under examination from the options of exposable body part;
when the to-be-exposed body part is a stitching body part, determining an exposure parameter set of the stitching body part, the exposure parameter set comprising multiple travel points that an X-ray source is capable of passing along at least two directions and exposure parameters about the X-ray source at the multiple travel points, and the at least two directions being capable of determining a two-dimensional surface or a three-dimensional space;
in response to an exposure instruction on the stitching body part, controlling, based on the exposure parameter set, the X-ray source to pass the multiple travel points along the at least two directions, and emit X-rays to the stitching body part at each of the travel points passed according to exposure parameters corresponding thereto;
controlling a detector to receive X-rays penetrating the stitching body part to obtain a plurality of digital X-ray images; and
stitching the plurality of digital X-ray images to obtain a radiograph of the stitching body part.

In some embodiments, determining an exposure parameter set of the stitching body part comprises:
acquiring a predetermined position relationship between the stitching body part and the detector, and determining the exposure parameter set of the stitching body part based on the predetermined position relationship; or,
acquiring a current position relationship between the stitching body part and the detector by a photographic apparatus, and determining the exposure parameter set of the stitching body part based on the current position relationship.

In some embodiments, determining the exposure parameter set of the stitching body part based on the current position relationship comprises:
displaying on the display interface or projecting by a projection apparatus the current position relationship between the stitching body part and the detector, and determining the exposure parameter set of the stitching body part based on a user operation.

In some embodiments, controlling, based on the exposure parameter set, the X-ray source to pass the multiple travel points along the at least two directions, and emit X-rays to the stitching body part at each of the travel points passed according to exposure parameters corresponding thereto comprises:
based on the exposure parameter set, controlling the X-ray source to pass one part of the multiple travel points along a first direction and emit the X-rays to the stitching body part at each of the travel points passed according to exposure parameters corresponding thereto, and controlling the X-ray source to pass the other part of the multiple travel points along a second direction and emit the X-rays to the stitching body part at each of the travel points passed according to exposure parameters corresponding thereto, the first and second directions forming the two-dimensional surface, wherein the at least two directions comprise the first and second directions, the first and second directions are both straightline directions or non straightline directions, or one of the first and second directions is a straightline direction and the other is a non straightline direction; or,
based on the exposure parameter set, controlling the X-ray source to pass a part of the multiple travel points along a first direction and emit the X-rays to the stitching body part at each of the travel points passed according to exposure parameters corresponding thereto, controlling the X-ray source to pass another part of the multiple travel points along a second direction and emit the X-rays to the stitching body part at each of the travel points passed according to exposure parameters corresponding thereto, and controlling the X-ray source to pass yet another part (such as the remaining part) of the multiple travel points along a third direction and emit the X-rays to the stitching body part at each of the travel points passed according to exposure parameters corresponding thereto, the first, second and third directions forming the three-dimensional space, wherein the at least two directions comprise the first, second and third directions, the first, second and third directions are all straightline directions or non straightline directions, or one of the first, second and third directions is a straightline direction or a non straightline direction.

In some embodiments, based on the exposure parameter set, controlling the X-ray source to pass one part of the multiple travel points along a first direction and emit the X-rays to the stitching body part at each of the travel points passed according to exposure parameters corresponding thereto, and controlling the X-ray source to pass the other part of the multiple travel points along a second direction and emit the X-rays to the stitching body part at each of the travel points passed according to exposure parameters corresponding thereto comprise:
based on the exposure parameter set, controlling the X-ray source to move along the first and second directions alternately to pass the multiple travel points.

In some embodiments, controlling a detector to receive X-rays penetrating the stitching body part to obtain a plurality of digital X-ray images comprises:
controlling the detector to move to a position corresponding to each travel point that the X-ray source passes and where the X-ray source emits X-rays and receive X-rays emitted by the X-ray source at said travel point and penetrating the stitching body part to obtain the plurality of digital X-ray images.

In some embodiments, the non straightline direction comprises at least one of a folded-line direction, a curve direction and a rotational direction.

In some embodiments, the first direction is one of a coronal axis direction, a sagittal axis direction and a vertical axis direction of the object under examination, the second direction is another one of a coronal axis direction, a sagittal axis direction and a vertical axis direction of the object under examination; or at least one of the first and second directions is a rotational direction around a target center corresponding to the X-ray source.

In some embodiments, controlling a detector to receive X-rays penetrating the stitching body part to obtain a plurality of digital X-ray images comprises:
controlling the detector to remain stationary relative to the X-ray source and receive X-rays emitted by the X-ray source at a travel point that the X-ray source passes and penetrating the stitching body part to obtain the plurality of digital X-ray images.

In some embodiments, determining an exposure parameter set of the stitching body part comprises:
acquiring at least one region of interest of the the stitching body part and determining the travel point related to the at least one region of interest, the at least one region of interest being located outside a stitching region of the stitching body part.

In some embodiments, the detector and the X-ray source are moved synchronously or successively.

In accordance with a second aspect, an digital X-ray imaging method provided in some embodiments may include:
determining a stitching body part of an object under examination;
determining a digital imaging mode of the stitching body part, the digital imaging mode comprising a first stitching mode and a second stitching mode, wherein the first stitching mode comprises controlling an X-ray source and/or a detector to move along one direction and expose the stitching body part to obtain a plurality of digital X-ray images and stitching the plurality of digital X-ray images to obtain a radiograph of the stitching body part, the second stitching mode comprises controlling an X-ray source and/or a detector to move along at least two directions and expose the stitching body part to obtain a plurality of digital X-ray images, and stitching the plurality of digital X-ray images to obtain a radiograph of the stitching body part, the at least two directions is capable of determining a two-dimensional surface or a three-dimensional space;
when the digital imaging mode is the first stitching mode, controlling the X-ray source and/or the detector to move along one direction and expose the stitching body part to obtain the plurality of digital X-ray images, and stitching the plurality of digital X-ray images to obtain the radiograph of the stitching body part; and
when the digital imaging mode is the second stitching mode, controlling the X-ray source and/or the detector to move along at least two directions and expose the stitching body part to obtain the plurality of digital X-ray images, and stitching the plurality of digital X-ray images to obtain the radiograph of the stitching body part.

In some embodiments, controlling the X-ray source and/or the detector to move along one direction and expose the stitching body part to obtain the plurality of digital X-ray images, and stitching the plurality of digital X-ray images to obtain the radiograph of the stitching body part comprise:
controlling the X-ray source to move to a plurality of first preset positions along one direction and controlling the detector to remain stationary relative to the X-ray source; when the X-ray source is moved to may of the first preset positions, controlling the X-ray source to emit X-rays to the stitching body part and controlling the detector to receive X-rays penetrating the stitching body part to obtain the plurality of digital X-ray images; and stitching the plurality of digital X-ray images to obtain the radiograph of the stitching body part; or,
controlling the detector to move to a plurality of second preset positions along one direction and controlling the X-ray source to remain stationary relative to the detector; when the detector is moved to any of the second preset positions, controlling the X-ray source to emit X-rays to the stitching body part and controlling the detector to receive X-rays penetrating the stitching body part to obtain the plurality of digital X-ray images; and stitching the plurality of digital X-ray images to obtain the radiograph of the stitching body part; or,
controlling the X-ray source to move to a plurality of first preset positions along one direction and controlling the detector to move along one direction to a plurality of second preset positions corresponding to the first preset positions; when the X-ray source and the detector are both moved to any of their respective preset positions, controlling the X-ray source to emit X-rays to the stitching body part and controlling the detector to receive X-rays penetrating the stitching body part to obtain the plurality of digital X-ray images; and stitching the plurality of digital X-ray images to obtain a radiograph of the stitching body part.

In some embodiments, controlling the X-ray source and/or the detector to move along at least two directions and expose the stitching body part to obtain the plurality of digital X-ray images, and stitching the plurality of digital X-ray images to obtain the radiograph of the stitching body part comprise:
controlling the X-ray source to move to a plurality of first preset positions along at least two directions and controlling the detector to remain stationary relative to the X-ray source; when the X-ray source is moved to any of the first preset positions, controlling the X-ray source to emit X-rays to the stitching body part and controlling the detector to receive X-rays penetrating the stitching body part to obtain the plurality of digital X-ray images; and stitching the plurality of digital X-ray images to obtain a radiograph of the stitching body part; or,
controlling the detector to move along at least two directions to a plurality of second preset positions and controlling the X-ray source to remain stationary relative to the detector; when the detector is moved to any of the second preset positions, controlling the X-ray source to emit X-rays to the stitching body part and controlling the detector to receive X-rays penetrating the stitching body part to obtain the plurality of digital X-ray images; and stitching the plurality of digital X-ray images to obtain a radiograph of the stitching body part; or,
controlling the X-ray source to move to a plurality of first preset positions along at least two directions and controlling the detector to move along at least two directions to a plurality of second preset positions corresponding to the first preset positions; when the X-ray source and the detector are both moved to any of their respective preset positions, controlling the X-ray source to emit X-rays to the stitching body part and controlling the detector to receive X-rays penetrating the stitching body part to obtain the plurality of digital X-ray images; and stitching the plurality of digital X-ray images to obtain a radiograph of the stitching body part.

In some embodiments, controlling the X-ray source to move to a plurality of first preset positions along at least two directions comprises:
controlling the X-ray source to pass one part of the plurality of first preset positions along a first direction and emit X-rays to the stitching body part at any of the first preset positions passed, and controlling the X-ray source to pass the other part of the plurality of first preset positions along a second direction and emit X-rays to the stitching body part at any of the first preset positions passed, the first and second directions forming the two-dimensional surface, wherein the at least two directions comprise the first and second directions, the first and second directions are both straightline directions or non straightline directions, or one of the first and second directions is a straightline direction and the other is a non straightline direction; or,
controlling the X-ray source to pass a part of the plurality of first preset positions along a first direction and emit X-rays to the stitching body part at any of the first preset positions passed, controlling the X-ray source to pass another part of plurality of first preset positions along a second direction and emit X-rays to the stitching body part at any of the first preset positions passed, and controlling the X-ray source to pass yet another part (such as the remaining part) of plurality of first preset positions along a third direction and emit X-rays to the stitching body part at any of the first preset positions passed, the first, second and third directions forming the three-dimensional space, wherein the at least two directions comprise the first, second and third directions, the first, second and third directions are all straightline directions or non straightline directions, or one of the first, second and third directions is a straightline direction or a non straightline direction.

In some embodiments, controlling the X-ray source to pass one part of the plurality of first preset positions along a first direction and emit X-rays to the stitching body part at any of the first preset positions passed, and controlling the X-ray source to pass the other part of the plurality of first preset positions along a second direction and emit X-rays to the stitching body part at any of the first preset positions passed comprise:
controlling the X-ray source to move along the first and second directions alternately to pass the plurality of first preset positions.

In some embodiments, controlling the detector to move along at least two directions to a plurality of second preset positions comprises:
controlling the detector to pass one part of the plurality of second preset positions along a first direction and receive X-rays penetrating the stitching body part at any of the second preset positions passed, and controlling the detector to pass the other part of the plurality of second preset positions along a second direction and receive X-rays penetrating the stitching body part at any of the second preset positions passed, the first and second directions forming the two-dimensional surface, wherein the at least two directions comprise the first and second directions, the the first and second directions are both straightline directions or non straightline directions, or one of the first and second directions is a straightline direction or a non straightline direction; or,
controlling the detector to pass a part of the plurality of second preset positions along a first direction and receive X-rays penetrating the stitching body part at any of the second preset positions passed, controlling the detector to pass another part of the plurality of second preset positions along a second direction and receive X-rays penetrating the stitching body part at any of the second preset positions passed, and controlling the detector to pass yet another part (such as the remaining part) of the plurality of second preset positions along a third direction and receive X-rays penetrating the stitching body part at any of the second preset positions passed, the first, second and third directions forming the three-dimensional space, wherein the at least two directions comprise the first, second and third directions, the first, second and third directions are all straightline directions or non straightline directions, or one of the first, second and third directions is a straightline direction or a non straightline direction.

In some embodiments, the first direction is one of a coronal axis direction, a sagittal axis direction and a vertical axis direction of the object under examination, and the second direction is another one of a coronal axis direction, a sagittal axis direction and a vertical axis direction of the object under examination; or at least one of the first and second directions is a rotational direction around a target center corresponding to the X-ray source.

In some embodiments, the non straightline direction comprises at least one of a folded-line direction, a curve direction and a rotational direction.

In some embodiments, determining a digital imaging mode of the stitching body part comprises:
displaying options of digital imaging mode on a display interface, and determining the digital imaging mode in response to a selection instruction on the options of digital imaging mode; or,
automatically determining the digital imaging mode according to the stitching body part.

In some embodiments, the method may further comprise:
switching among digital imaging modes in response to a triggering of a mode switching button.

In accordance with a third aspect, an digital X-ray imaging method provided in some embodiments may include:
determining a stitching body part of an object under examination;
obtaining a plurality of digital X-ray images of the stitching body part, wherein the plurality of digital X-ray images are obtained while an X-ray source and/or a detector are/is controlled to move along at least two directions and the stitching body part is exposed, the at least two directions are capable of determining a two-dimensional surface or a three-dimensional space; and
stitching the plurality of digital X-ray images to obtain a radiograph of the stitching body part.

In some embodiments, obtaining a plurality of digital X-ray images of the stitching body part comprises:
obtaining the plurality of digital X-ray images based on historical images; or,
under a current digital imaging mode, obtaining the plurality of digital X-ray images while the X-ray source and/or the detector are/is controlled to move along at least two directions and the stitching body part is exposed.

In some embodiments, obtaining the plurality of digital X-ray images while the X-ray source and/or the detector are/is controlled to move along at least two directions and the stitching body part is exposed comprises:
controlling the X-ray source to move to a plurality of first preset positions along at least two directions and controlling the detector to remain stationary relative to the X-ray source, and when the X-ray source is moved to any of the first preset positions, obtaining the plurality of digital X-ray images while the X-ray source is controlled to emit X-rays to the stitching body part and the detector is controlled to receive X-rays penetrating the stitching body part; or,
controlling the detector to move to a plurality of second preset positions along at least two directions and controlling the X-ray source to remain stationary relative to the detector, when the detector is moved to any of the second preset positions, obtaining the plurality of digital X-ray images while the X-ray source is controlled to emit X-rays to the stitching body part and the detector is controlled to receive X-rays penetrating the stitching body part; or,
controlling the X-ray source to move to a plurality of first preset positions along at least two directions and controlling the detector to move to second preset positions corresponding to the plurality of first preset positions along at least two directions; when the X-ray source and the detector are both moved to any of their respective preset positions, obtaining the plurality of digital X-ray images while the X-ray source is controlled to emit X-rays to the stitching body part and the detector is controlled to receive X-rays penetrating the stitching body part; and stitching the plurality of digital X-ray images to obtain a radiograph of the stitching body part.

In some embodiments, controlling the X-ray source to move to a plurality of first preset positions along at least two directions comprises:
controlling the X-ray source to pass one part of the plurality of first preset positions along a first direction and emit X-rays to the stitching body part at any of the first preset positions passed, and controlling the X-ray source to pass the other part of the plurality of first preset positions along a second direction and emit X-rays to the stitching body part at any of the first preset positions passed, the first and second directions forming the two-dimensional surface, wherein the at least two directions comprises the first and second directions, the first and second directions are both straightline directions or non straightline directions, or one of the first and second directions is a straightline direction or a non straightline direction; or,
controlling the X-ray source to pass a part of the plurality of first preset positions along a first direction and emit X-rays to the stitching body part at any of the first preset positions passed, controlling the X-ray source to pass another part of the plurality of first preset positions along a second direction and emit X-rays to the stitching body part at any of the first preset positions passed, and controlling the X-ray source to pass yet another part (such as the remaining part) of the plurality of first preset positions along a third direction and emit X-rays to the stitching body part at any of the first preset positions passed, the first, second and third directions forming three-dimensional space, wherein the at least two directions comprise the first, second and third directions, the first, second and third directions are all straightline directions or non straightline directions, or one of the first, second and third directions is a straightline direction or a non straightline direction.

In some embodiments, controlling the X-ray source to pass one part of the plurality of first preset positions along a first direction and emit X-rays to the stitching body part at any of the first preset positions passed, and controlling the X-ray source to pass the other part of the plurality of first preset positions along a second direction and emit X-rays to the stitching body part at any of the first preset positions passed comprise:
controlling the X-ray source to move along the first and second directions alternately to pass the plurality of first preset positions.

In some embodiments, controlling the detector to move to a plurality of second preset positions along at least two directions comprises:
controlling the detector to pass one part of the plurality of second preset positions along a first direction and receive X-rays penetrating the stitching body part at any of the second preset positions passed, and controlling the detector to pass the other part of the plurality of second preset positions along a second direction and receive X-rays penetrating the stitching body part at any of the second preset positions passed, the first and second directions forming the two-dimensional surface, wherein the at least two directions comprise the first and second directions, the first and second directions are both straightline directions or non straightline directions, or one of the first and second directions is a straightline direction or a non straightline direction; or,
controlling the detector to pass a part of the plurality of second preset positions along a first direction and receive X-rays penetrating the stitching body part at any of the second preset positions passed, controlling the detector to pass another part of the plurality of second preset positions along a second direction and receive X-rays penetrating the stitching body part at any of the second preset positions passed, and controlling the detector to pass yet another part (such as the remaining part) of the plurality of second preset positions along a third direction and receive X-rays penetrating the stitching body part at any of the second preset positions passed, the first, second and third directions forming the three-dimensional space, wherein the at least two directions comprise the first, second and third directions, the first, second and third directions are all straightline directions or non straightline directions, or one of the first, second and third directions is a straightline direction or a non straightline direction.

In some embodiments, the first direction is one of a coronal axis direction, a sagittal axis direction and a vertical axis direction of the object under examination, and the second direction is another one of a coronal axis direction, a sagittal axis direction and a vertical axis direction of the object under examination; or at least one of the first and second directions is a rotational direction around a target center corresponding to the X-ray source.

In some embodiments, the non straightline direction comprises at least one of a folded-line direction, a curve direction and a rotational direction.

In some embodiments, stitching the plurality of digital X-ray images to obtain a radiograph of the stitching body part comprises:
stitching the plurality of digital X-ray images in at least two stitching directions to obtain a radiograph of the stitching body part.

In some embodiments, stitching the plurality of digital X-ray images in at least two stitching directions to obtain a radiograph of the stitching body part comprises:
stitching at least two digital X-ray images from the plurality of digital X-ray images in a first stitching direction to obtain an intermediate stitching image, said at least two digital X-ray images being obtained while the X-ray source and/or the detector pass/passes a preset position along the first direction for exposure; and stitching the intermediate stitching image and at least one digital X-ray image from the plurality of digital X-ray images in a second stitching direction to obtain the radiograph of the stitching body part, said at least one digital X-ray image being obtained while the X-ray source and/or the detector pass/passes a preset position along the second direction for exposure; or,
stitching at least two digital X-ray images from the plurality of digital X-ray images in a second stitching direction to obtain an intermediate stitching image, said at least two digital X-ray images being obtained while the X-ray source and/or the detector pass/passes at a preset position along the second direction for exposure; and stitching the intermediate stitching image and at least one digital X-ray image from the plurality of digital X-ray images in a first stitching direction to obtain a radiograph of the stitching body part, said at least one digital X-ray image being obtained while the X-ray source and/or the detector pass/passes at a preset position along the first direction for exposure; or,
stitching at least three digital X-ray images from the plurality of digital X-ray images in a first stitching direction to obtain at least two intermediate stitching images, said at least three digital X-ray images being obtained while the X-ray source and/or the detector pass/passes at a preset position along the first direction for exposure; and stitching the at least two intermediate stitching images in a second stitching direction to obtain a radiograph of the stitching body part; or,
stitching at least three digital X-ray images from the plurality of digital X-ray images in a second stitching direction to obtain at least two intermediate stitching images, said at least three digital X-ray images being obtained while the X-ray source and/or the detector pass/passes at a preset position along the second direction for exposure; and stitching the at least two intermediate stitching images in a first stitching direction to obtain a radiograph of the stitching body part.

In accordance with a fourth aspect, an digital X-ray imaging method provided in some embodiments may include:
determining a stitching body part of an object under examination;
obtaining a plurality of digital X-ray images of the stitching body part, wherein the plurality of digital X-ray images are obtained while an X-ray source and/or a detector are/is controlled to move along a coronal axis direction of the object under examination and the stitching body part is exposed; and
stitching the plurality of digital X-ray images to obtain a radiograph of the stitching body part.

In some embodiments, obtaining a plurality of digital X-ray images of the stitching body part comprises:
obtaining the plurality of digital X-ray images based on historical images; or,
under a current digital imaging mode, obtaining the plurality of digital X-ray images while the X-ray source and/or the detector are/is controlled to move along the coronal axis direction of the object under examination and the stitching body part is exposed.

In some embodiments, obtaining the plurality of digital X-ray images while the X-ray source and/or the detector are/is controlled to move along the coronal axis direction of the object under examination and the stitching body part is exposed comprises:
controlling the X-ray source to move to a plurality of first preset positions along the coronal axis direction of the object under examination and controlling the detector to remain stationary relative to the X-ray source, and when the X-ray source is moved to any of the first preset positions, obtaining the plurality of digital X-ray images while the X-ray source is controlled to emit X-rays to the stitching body part and the detector is controlled to receive X-rays penetrating the stitching body part; or,
controlling the detector to move to a plurality of second preset positions along the coronal axis direction of the object under examination and controlling the X-ray source to remain stationary relative to the detector, and when the detector is moved to any of the second preset positions, obtaining the plurality of digital X-ray images while the X-ray source is controlled to emit X-rays to the stitching body part and the detector is controlled to receive X-rays penetrating the stitching body part; or,
controlling the X-ray source to move to a plurality of first preset positions along the coronal axis direction of the object under examination and controlling the detector to move to second preset positions corresponding to the plurality of first preset positions along the coronal axis direction of the object under examination, and when the X-ray source and the detector are both moved to any of their respective preset positions, obtaining the plurality of digital X-ray images while the X-ray source is controlled to emit X-rays to the stitching body part and the detector is controlled to receive X-rays penetrating the stitching body part.

In accordance with a fifth aspect, an digital X-ray imaging method provided in some embodiments may include:
determining a stitching body part of an object under examination;
obtaining a plurality of digital X-ray images of the stitching body part, wherein the plurality of digital X-ray images are obtained while an X-ray source and/or a detector are/is controlled to move along at least one non straightline direction and the stitching body part is exposed; and
stitching the plurality of digital X-ray images to obtain a radiograph of the stitching body part.

In some embodiments, obtaining a plurality of digital X-ray images of the stitching body part comprises:
obtaining the plurality of digital X-ray images based on historical images; or,
under a current digital imaging mode, obtaining the plurality of digital X-ray images while the X-ray source and/or the detector are/is controlled to move along at least one non straightline direction and the stitching body part is exposed.

In some embodiments, obtaining the plurality of digital X-ray images while the X-ray source and/or the detector are/is controlled to move along at least one non straightline direction and the stitching body part is exposed comprises:
controlling the X-ray source to move to a plurality of first preset positions along at least one non straightline direction and controlling the detector to remain stationary relative to the X-ray source, and when the X-ray source is moved to any of the first preset positions, obtaining the plurality of digital X-ray images while the X-ray source is controlled to emit X-rays to the stitching body part and the detector is controlled to receive X-rays penetrating the stitching body part; or,
controlling the detector to move to a plurality of second preset positions along at least one non straightline direction and controlling the X-ray source to remain stationary relative to the detector, and when the detector is moved to any of the second preset positions, obtaining the plurality of digital X-ray images while the X-ray source is controlled to emit X-rays to the stitching body part and the detector is controlled to receive X-rays penetrating the stitching body part; or,
controlling the X-ray source to move to a plurality of first preset positions along at least one non straightline direction and controlling the detector to move to second preset positions corresponding to the plurality of first preset positions along at least one non straightline direction, and when the X-ray source and the detector are both moved to any of their respective preset positions, obtaining the plurality of digital X-ray images while the X-ray source is controlled to emit X-rays to the stitching body part and the detector is controlled to receive X-rays penetrating the stitching body part.

In some embodiments, the non straightline direction comprises at least one of a folded-line direction, a curve direction and a rotational direction.

In accordance with a sixth aspect, a digital X-ray imaging apparatus provided in some embodiments may comprise:
an X-ray source configured to emit X-rays to an object under examination;
a detector configured to receive the X-rays penetrating the object under examination; and
a processor configured to execute the method according to any one of the embodiments in the present disclosure.

With the digital X-ray imaging methods and the digital X-ray imaging apparatus mentioned in some embodiments above, when the to-be-exposed body part is the stitching body part, the exposure parameter set of the stitching body part is determined, the exposure parameter set may include a plurality of travel points that an X-ray source is capable of passing along at least two directions and exposure parameters about the X-ray source at multiple travel points, the at least two directions are capable of determining a two-dimensional surface or a three-dimensional space, then, based on the exposure parameter set, the X-ray source is controlled to pass the multiple travel points along the at least two directions and emit X-rays to the stitching body part at each of the travel points passed according to exposure parameters corresponding thereto and the detector is controlled to receive the X-rays penetrating the stitching body part to obtain a plurality of digital X-ray images, and the radiograph of the stitching body part is obtained by stitching the plurality of digital X-ray images. Therefore, radiographing and stitching of the stitching body part can be achieved by the present disclosure.

### BRIEF DESCRIPTION OF THE DRAWINGS

FIG. 1 is a schematic diagram of three standard planes and axes corresponding to the anatomical posture of a human body;
FIG. 2(a) is a schematic diagram of the structure of a digital X-ray imaging apparatus in some embodiments;
FIG. 2(b) is another schematic diagram of the structure of a digital X-ray imaging apparatus in some embodiments;
FIG. 3 is a schematic diagram of the structure of an X-ray source in some embodiments;
FIG. 4 is a schematic diagram of the structure of a digital X-ray imaging apparatus in some embodiments;
FIG. 5 is a schematic diagram of the structure of a detector in some embodiments;
FIG. 6 is a schematic diagram of the structure of a digital X-ray imaging apparatus in some embodiments;
FIG. 7 is a schematic diagram showing the stitching body part of an object under examination being a lower limb in some embodiments;
FIGs. 8(a) to 8(e) are five schematic diagrams of intermediate processes of performing digital X-ray imaging on the stitching body part of an object under examination being a lower limb in some embodiments;
FIGs. 9(a) to 9(c) are three schematic diagrams of intermediate processes of performing digital X-ray imaging on the stitching body part of an object under examination being a lower limb in some embodiments;
FIGs. 10(a) to 10(e) are five schematic diagrams of regions that need to be imaged when the stitching body part of an object under examination is a chest;
FIGs. 11(a) and 11(b) are two schematic diagrams of performing digital X-ray imaging on the stitching body part of an object under examination being the chest shown in FIG. 10(e);
FIGs. 12(a) to 12(c) are three schematic diagrams of performing digital X-ray imaging on the stitching body part of an object under examination being the chest shown in FIG. 10(e);
FIGs. 13(a) and 13(b) are two schematic diagrams of intermediate processes of performing digital X-ray imaging on the stitching body part of an object under examination being the chest shown in FIG. 10(e);
FIG. 14 is a schematic diagram of displaying options of an exposable body part of an object under examination on a display interface in some embodiments;
FIG. 15 is a schematic diagram of the structure of a digital X-ray imaging apparatus in some embodiments;
FIG. 16 is a schematic diagram of the structure of a digital X-ray imaging apparatus in some embodiments;
FIG. 17 is a schematic diagram of selecting three regions of interest on a first image in some embodiments;
FIG. 18 is a schematic diagram of the exposure parameter set obtained after selecting a region of interest based on a first image in some embodiments;
FIG. 19 is a flow chart of a digital X-ray imaging method in some embodiments;
FIG. 20 is a flow chart of a digital X-ray imaging method in some embodiments;
FIG. 21 is a flow chart of a digital X-ray imaging method in some embodiments;
FIG. 22 is a flow chart of a digital X-ray imaging method in some embodiments; and
FIG. 23 is a flow chart of a digital X-ray imaging method in some embodiments.

### DETAILED DESCRIPTION

The present disclosure is further described in detail below through specific embodiments in combination with the drawings, wherein, similar elements in different embodiments adopt associated similar element labels. In the following embodiments, many details are described in order to make the application be better understood. However, those skilled in the art can easily realize that some features can be omitted in different cases or can be replaced by other elements, materials and methods. In some cases, some operations related to the present disclosure are not shown or described in the specification in order to avoid the core part of the present disclosure being overwhelmed by excessive descriptions, and for those skilled in the art, it is not necessary to describe these relevant operations in detail, they can completely understand the relevant operations according to the description in the specification and the general technical knowledge of the field.

In addition, the features, operations or characteristics described in the specification may be combined in any appropriate manner to form various embodiments. At the same time, the steps or actions described in the method may be sequenced or adjusted in a manner apparent to those skilled in the art. Therefore, the sequences in the specification and the drawings are intended to clearly describe an embodiment and are not meant to be a required sequence unless it is indicated otherwise that a sequence must be followed.

The serial numbers assigned to the parts in the present disclosure, such as "first", "second", etc., are only used to distinguish the described objects, and do not have any sequential or technical meaning. The terms "connect" and "couple" as mentioned in the present disclosure, unless otherwise specified, include direct and indirect connection (coupling).

Several important terms are explained firstly.

A stitching body part mentioned in some embodiments of the present disclosure may refer to a tissue part of an object under examination, wherein the tissue part may need to be radiographed many times during X-ray imaging, and a radiograph of the tissue part may be obtained by stitching a plurality of digital X-ray images. For example, the stitching body parts may be a chest, an abdomen or a lower limb. It shall be understood that the stitching body parts does not refer to a certain posture of the object under examination.

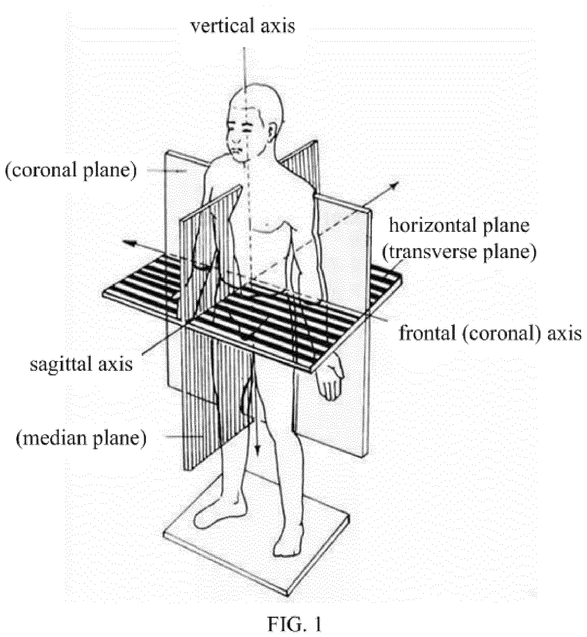

Three axes of a human body or a object under examination, namely a coronal axis, a sagittal axis, and a vertical axis, are involved herein. FIG. 1 schematically shows three standard planes and three standard axes corresponding to an anatomical posture. When describing the azimuth axis or plane of the human body, terms such as upper, lower, front, rear, left and right it refers to are all based on the orientation of the anatomical posture of a person being described; wherein the anatomical posture is defined as follows: the person is upright with legs closed together, both upper limbs hanging down, eyes looking straight ahead, and both palms and both feet pointing forward. Specifically speaking, the vertical axis may refer to a longitudinal axis that runs through the human body or part thereof in an up-down direction and is perpendicular to a horizontal plane; the sagittal axis may refer to a horizontal axis that runs through the human body or part thereof in a front-rear direction and is perpendicular to a coronal plane; and the coronal axis, also known as a frontal axis, is a horizontal axis that runs through the human body or part thereof in a left-right direction and is perpendicular to a sagittal plane. There are also three sections of the human body and part thereof, including: the coronal plane (also known as a frontal plane) dividing the human body or part thereof vertically into two parts (front and rear parts) and being perpendicular to the sagittal axis; the sagittal plane dividing the human body or part thereof vertically into two parts (left and right parts) and being perpendicular to the coronal axis; and the horizontal plane (also known as a transverse plane) dividing the human body or part thereof into two parts (upper and lower parts) and being perpendicular to the vertical axis. The vertical plane passing through the sagittal suture within cranial bones may be referred to as a median sagittal plane or median plane, whereas other sagittal planes are parallel to the median sagittal plane. In some embodiments, the coronal axis, sagittal axis and vertical axis may be straight or approximate straight lines.

In some embodiments of the present disclosure, "multiple" may refer to at least two, and "a plurality of" may refer to at least two.

In some embodiments of the present disclosure, "at least two directions being capable of determining a two-dimensional surface or a three-dimensional space" may refer to allowing an X-ray source and/or a detector to move along at least two directions, wherein the at least two directions may be different directions of motion. The at least two directions may be directions of motion along straight lines or curves, and the straight lines or curves corresponding to the at least two directions may be intersected directly or via their extension lines, thereby defining a two-dimensional surface. The two-dimensional surface may be a two-dimensional planar surface or a two-dimensional curved surface. The two parallel or approximately parallel lines may be regarded as one direction; for example, two parallel lines from left to right and/or from right to left may be regarded as one direction, and two parallel lines from top to bottom and/or from bottom to top may be regarded as one direction. For another example, lines parallel or approximately parallel to the X-axis may be considered as one direction, lines parallel or approximately parallel to the Y-axis may be considered as one direction, and lines parallel or approximately parallel to the Z-axis may be considered as one direction.

If the two-dimensional surface is a two-dimensional planar surface, it may be determined by intersection of at least two curves and/or lines. For example, a line from left to right is one direction, another line from top to bottom is another direction, and a two-dimensional planar surface is determined by direct intersection of these two directions or intersection of their extension.

If the two-dimensional surface is a two-dimensional curved surface, the two-dimensional curved surface may be determined by at least two curves or by a curve and a straight line. For example, a curve formed by moving around a certain center point or along an arc is a one-dimensional space, and further linear or curved motion may be carried out along a trajectory that can intersect (directly or extended) with the curve in space in this one-dimensional space to determine a two-dimensional curved surface, such as a spherical or cylindrical surface.

In addition, the at least two directions can determine a three-dimensional space which may include at least a two-dimensional surface and a one-dimensional line. The one-dimensional line may be a straight line or a curve, and the two-dimensional surface may be a curved surface or a planar surface. The determination of the two-dimensional surface is as described above. In an example, the X-ray source and/or the detector may be moved along three straight lines, the X-axis, the Y-axis and the Z-axis, in a three-dimensional space. A line parallel or approximately parallel to the X-axis may be considered as a direction, another line parallel or approximately parallel to the Y-axis may be considered as another direction, and yet another line parallel or approximately parallel to the Z-axis may be considered as yet another direction, which may determine a three-dimensional space.

In some embodiments of the present disclosure, the "at least two stitching directions" may refer to, after obtaining a plurality of digital X-ray images, stitching a plurality of digital X-ray images in at least two stitching directions to obtain a radiograph of the stitching body part, wherein the at least two stitching directions are different, the at least two stitching directions may be of straight lines or curves, and the straight lines or curves corresponding to the at least two stitching directions may be intersected directly or via their extension lines. Two parallel or approximately parallel lines may be considered as a stitching direction. For example, two parallel lines from left to right and/or from right to left may be considered as a stitching direction, while two parallel lines from top to bottom and/or from bottom to top may be considered as a stitching direction. For another example, straight lines parallel or approximately parallel to the X-axis may be considered as a stitching direction, straight lines parallel or approximately parallel to the Y-axis may be considered as another stitching direction, and straight lines parallel or approximately parallel to the Z-axis may be considered as yet another stitching direction. The stitching process may refer to, for example, stitching adjacent images from left to right and then from top to bottom, wherein stitching from left to right may refer to a stitching direction, and stitching from top to bottom may refer to another stitching direction.

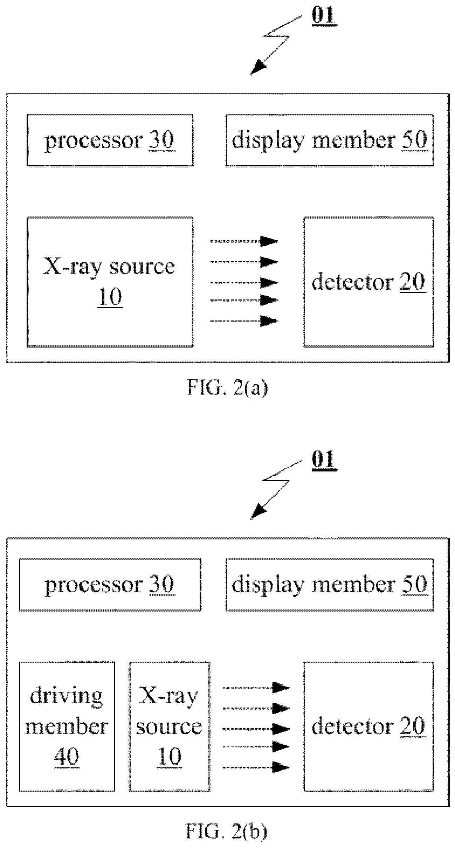

Referring to FIG. 2, in some embodiments, the digital X-ray imaging apparatus 01 may include an X-ray source 10, a detector 20 and a processor 30. Additionally or alternatively, referring to FIG. 2(b), in some embodiment, the digital X-ray imaging apparatus 01 may further include one or more of a driving member 40 and a display member 50. This will be illustrated in detailed below.

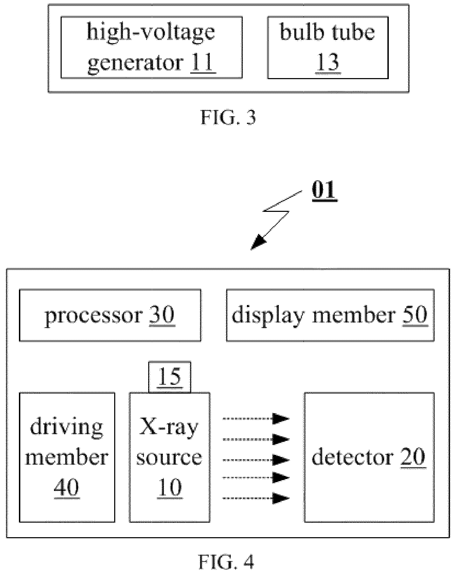

The X-ray source 10 may be configured to emit X-rays towards an object under examination, such as a to-be-exposed body part. In some embodiments, the X-ray source 10 may be arranged relative to the detector 20 during operation. Referring to FIG. 3, the X-ray source 10 in some embodiments may include a high-voltage generator 11 and a bulb tube 13. The high-voltage generator 11 may be configured to provide a voltage, such as a kilovolt high voltage, to the bulb tube 13; and the bulb tube 13 may be configured to bombard electrons onto a target surface under the provided voltage from the high-voltage generator 11 to generate radiation including X-rays.

In some embodiments, referring to FIG. 4, the X-ray source 10 is provided with an X-ray beam limiter 15 configured to determine or simulate the radiating area of the X-ray source 10. The area irradiated by the X-ray beam limiter 15 may be referred to as an irradiation field. In the present disclosure, the motion of the X-ray source controlled may mainly be achieved by the movement of a head piece. In some embodiments, the movement of the head piece allows the irradiation field corresponding to the X-ray source to move accordingly, which is equivalent to the radiation field also moving in at least one direction when it moves. The movement of the radiation field is to maintain alignment with the detector.

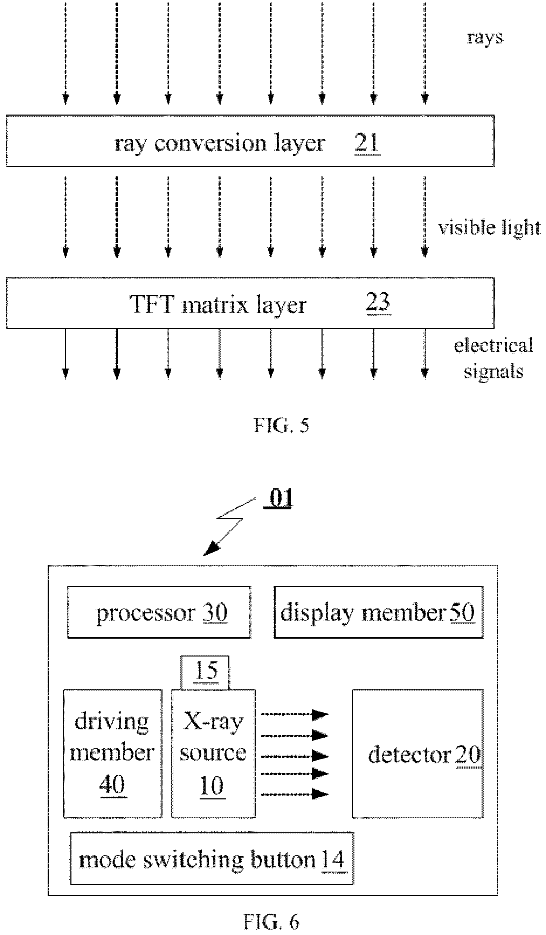

The detector 20 may be configured to receive X-rays penetrating the object under examination, such as the to-be-examined body position thereof. The detector 20 is a crucial component of the digital X-ray imaging apparatus 01 and plays a decisive role in ensuring high-quality imaging. It receives and converts the rays into electrical signals, thereby completing the acquisition of image information. Referring to FIG. 5, the detector 20 in some embodiments may include a ray conversion layer 21 and a TFT matrix layer 23. The ray conversion layer 21 may be configured to convert the rays into visible light. The ray conversion layer 21 may typically include a scintillation layer or a fluorescent layer for converting the rays into visible light. Taking the scintillation layer as an example, it may generally be made of scintillation materials, such as cesium iodide (CsI) or gadolinium oxysulfide (GOS). The TFT matrix layer 23 may be configured to sense the visible light converted by the ray conversion layer 21 and convert the visible light into electrical signals for acquiring image information.

In some embodiments, the detector 20 may be a flat panel detector.

As can be seen, the detector 20 is a ray-receiving unit in the digital X-ray imaging apparatus 01. The X-ray source 10 generates and emits X-rays which penetrate the to-be-exposed body part of the object under examination and undergo attenuation, and the attenuated X-rays can be received by the detector 20; subsequently, the detector 20 receives the X-rays penetrated the to-be-exposed body part of the object under examination for imaging. Specifically, the detector 20 captures and convert the X-rays into visible light before transforming them into electrical signals.

In some embodiments, the digital X-ray imaging apparatus 01 may include one or more detectors 20.

In some embodiments, the driving member 40 may be configured to drive the X-ray source 10 to move, such as to displace, for example to turn or rotate. In some examples, when the driving member 40 drives the X-ray source 10 to turn or rotate, it may turn or rotate around a target center corresponding to the X-ray source. The target center may be, for example, the focus of a ball tube 13 or a nearby point.

In some embodiments, the X-ray source 10 may be controlled manually by a user to move, such as to displace, for example to turn or rotate. In some examples, when the X-ray source 10 is controlled manually by the user to turn or rotate, it may turn or rotate around a target center corresponding to the X-ray source. The target center may be, for example, the focus of the ball tube 13 or a nearby point.

In some examples, the movement of the X-ray source 10 controlled manually by a user may refer to applying a force directly by the user to the X-ray source 10 to make it move under the force. For example, the user may drag the X-ray source 10 by hand to make it move.

In some examples, the movement of the X-ray source 10 controlled manually by a user may refer to sending an instruction by the user via a mouse, a keyboard, a touch screen or a voice input component to the digital X-ray imaging apparatus 01 to make the X-ray source 10 move; then, the X-ray source 10 may be driven to move accordingly by the processor 30 through the driving member 40 after the digital X-ray imaging apparatus 01 receives the instruction.

In this regard, the X-ray source 10 may be arranged within the digital X-ray imaging apparatus 01 in a turnable or rotatable manner in some examples, wherein the point around which the X-ray source 10 turns/rotates may be the focus of the ball tube 13 or a nearby point.

In some embodiments, the driving member 40 may be configured to drive the detector 20 to move, such as to displace, for example to turn or rotate.

In some embodiments, the detector 20 may be controlled manually by a user to move, such as to displace, for example to turn or rotate.

In some examples, the movement of the detector 20 controlled manually by a user may refer to applying a force directly by the user to the detector 20 to make it move under the force. For example, the user may drag the detector 20 by hand to make it move.

In some examples, the movement of the detector 20 controlled manually by a user may refer to sending an instruction by the user via a mouse, a keyboard, a touch screen or a voice input component to the digital X-ray imaging apparatus 01 to make the detector 20 move; then, the detector 20 may be driven to move accordingly by the processor 30 through the driving member 40 after the digital X-ray imaging apparatus 01 receives the instruction.

In some embodiments, the driving member 40 may be configured to drive the X-ray source 10 and the detector 20 to move, such as to displace, for example to turn or rotate. In some examples, the driving member 40 may be configured to drive the X-ray source 10 and the detector 20 to move synchronously.

The display member 50 may be configured to display, for example intermediate or final imaging results, and for example, a user interface, which will be further described below.

In some embodiments, the processor 30 may include but be not limited to a central processing unit (CPU), a micro controller unit (MCU), a field programmable gate array (FPGA), a digital signal processing (DSP) and other devices used to interpret computer instructions and process data in computer software.

In some embodiments, the digital X-ray imaging apparatus 01 involved herein may also be used to execute and implement the method or one or more steps described in any embodiment herein. For example, the processor 30 may be used to execute and implement the method or one or more steps described in any embodiment herein.

In some embodiments, the processor 30 may obtain a plurality of digital X-ray images of the stitching body part, and stitch the plurality of digital X-ray images to obtain the radiograph of the stitching body part. Before this, the stitching body part of the object under examination may also be determined firstly. Accordingly, in some embodiments, the processor 30 may determine the stitching body part of the object under examination, obtain a plurality of digital X-ray images of the stitching body part, and stitch the plurality of digital X-ray images to obtain the radiograph of the stitching body part.

In some examples, the plurality of digital X-ray images mentioned above may be obtained while the X-ray source 10 and/or the detector 20 are/is controlled to move along one direction and the stitching body part is exposed. For example, the plurality of digital X-ray images may be obtained while the X-ray source 10 and/or the detector 20 are/is controlled to move along the coronal axis direction of the object under examination and the stitching body part is exposed.

The X-ray source 10 being controlled to move along one direction herein may refer to: automatically controlling the X-ray source 10 by the processor 30 to move along one direction, or manually controlling the X-ray source 10 by a user to move along one direction. The detector 20 being controlled to move along one direction herein may refer to: automatically controlling the detector 20 by the processor 30 to move along one direction, or manually controlling the detector 20 by a user to move along one direction.

In some examples, the plurality of digital X-ray images mentioned above may be obtained while the X-ray source 10 and/or the detector 20 are/is controlled to move along at least two directions and the stitching body part is exposed, wherein the at least two directions are capable of determining a two-dimensional surface or a three-dimensional space. The two-dimensional surface herein may be a planar surface or a curved surface which may be for example a cylindrical surface or an arc surface.

The X-ray source 10 and/or the detector 20 being controlled to move along at least two directions herein may refer to: automatically controlling the X-ray source 10 by the processor 30 to move and automatically controlling the detector 20 by the processor 30 to move; or, automatically controlling the X-ray source 10 by the processor 30 to move and manually controlling the detector 20 by a user to move; or, manually controlling the X-ray source 10 by a user to move and automatically controlling the detector 20 by the processor 30 to move; or, manually controlling the X-ray source 10 by a user to move and manually controlling the detector 20 by a user to move.

In some embodiments, the processor 30 obtaining the plurality of digital X-ray images of the stitching body part may include: obtaining the plurality of digital X-ray images based on historical images. The historical images here may refer to digital X-ray images that have been exposed and stored in advance.

In some embodiments, the processor 30 may select a plurality of digital X-ray images from the stored historical images in response to an image selection instruction. For example, the display member 50 is capable of displaying the stored historical images from which a plurality of digital X-ray images required may be selected by a user via a tool such as a mouse; and in response to a stitching instruction, the processor 30 may stitch the selected plurality of digital X-ray images to obtain a stitched radiograph, i.e., the radiograph of the stitching body part.

In some embodiments, the digital X-ray imaging apparatus 01 may be provided with one or more digital imaging modes. The digital imaging modes may be digital imaging modes of the stitching body part. In some examples, the digital imaging modes may include at least one of a first stitching mode and a second stitching mode, that is, the digital X-ray imaging apparatus 01 may be provided with at least one of the first stitching mode and the second stitching mode.

In some examples, the display member 50 may display options of digital imaging mode on the display interface; and the processor 30 may determine a digital imaging mode in response to the selection instruction on the options of digital imaging mode.

In some examples, the processor 30 may automatically determine the digital imaging mode according to the stitching body part. For example, when the stitching body part is a first type of stitching body part, determining its corresponding digital imaging mode may be a first stitching mode; and when the stitching body part is a second type of stitching body part, determining its corresponding digital imaging mode may be a second stitching mode. The first type of stitching body part and the second type of stitching body part may be predetermined.

In some examples, referring to FIG. 6, a mode switching button 14 may be introduced. The mode switching button 14 may be a physical button or a virtual button, such as an option of mode switching, the option may be represented such as by text or icon. In response to the triggering of the mode switching button 14, the processor 30 may control switching among the digital imaging modes, such as between the first and second stitching modes.

The first stitching mode is described below.

In some embodiments, the first stitching mode may include obtaining a plurality of digital X-ray images while the X-ray source 10 and/or the detector 20 are/is controlled to move along one direction and the stitching body part is exposed, and stitching the plurality of digital X-ray images to obtain the radiograph of the stitching body part, that is, the plurality of digital X-ray images are used to stitch to obtain the radiograph of the stitching body part. For example, the first stitching mode may include obtaining a plurality of digital X-ray images while the X-ray source 10 and/or the detector 20 are/is controlled to move along the coronal axis direction of the object under examination and the stitching body part is exposed, wherein the plurality of digital X-ray images are used to stitch to obtain the radiograph of the stitching body part.

Accordingly, an imaging process may be as follows:
The processor 30 may determine a stitching body part of an object under examination, determine the digital imaging mode of the stitching body part, when the digital imaging mode is the first stitching mode, obtain a plurality of digital X-ray images while the X-ray source 10 and/or the detector 20 are/is controlled to move along one direction (e.g. the coronal axis direction of the object under examination) and the stitching body part is exposed, and stitch the plurality of digital X-ray images to obtain the radiograph of the stitching body part. That is, the plurality of digital X-ray images are used to stitch to obtain the radiograph of the stitching body part.

The X-ray source 10 being controlled to move along one direction herein may refer to: automatically controlling the X-ray source 10 by the processor 30 to move along one direction, or manually controlling the X-ray source 10 by a user to move along one direction. The detector 20 being controlled to move along one direction herein may refer to: automatically controlling the detector 20 by the processor 30 to move along one direction, or manually controlling the detector 20 by a user to move along one direction.

In some embodiments, the X-ray source 10 is controlled to move to a plurality of first preset positions along one direction (e.g. the coronal axis direction of the object under examination), and when the X-ray source 10 is moved to any of the first preset positions, the X-ray source 10 is controlled to emit X-rays to the stitching body part, and the detector 20 is controlled to receive the X-rays penetrating the stitching body part, thereby obtaining the plurality of digital X-ray images. The plurality of first preset positions involved here may be characterized by multiple specific positions; in this regard, the X-ray source 10 is controlled to move to the multiple specific positions along one direction, and emit X-rays to the stitching body part at the multiple specific positions. Alternatively or additionally, the plurality of first preset positions involved here may be characterized by a specific motion track; in this regard, the X-ray source 10 is controlled to move to a plurality of first preset positions along one direction, that is, the X-ray source 10 is controlled to move along the motion track and emit X-rays to the stitching body part at a plurality of positions on the motion track.

The X-ray source 10 being controlled to move to a plurality of first preset positions along one direction may refer to: automatically controlling the X-ray source 10 by the processor 30 to move to a plurality of first preset positions along one direction, or manually controlling the X-ray source 10 by a user to move to a plurality of first preset positions along one direction.

In some embodiments, said one direction mentioned above may be a straightline direction or a non straightline direction. In some embodiments, the non straightline directions may include at least one of a folded-line direction, a curve direction and a rotational direction. In some examples, the curve direction may be such as a direction of an arc.

In some embodiments, said one direction mentioned above may be one of a coronal axis direction, a sagittal axis direction and a vertical axis direction of the object under examination.

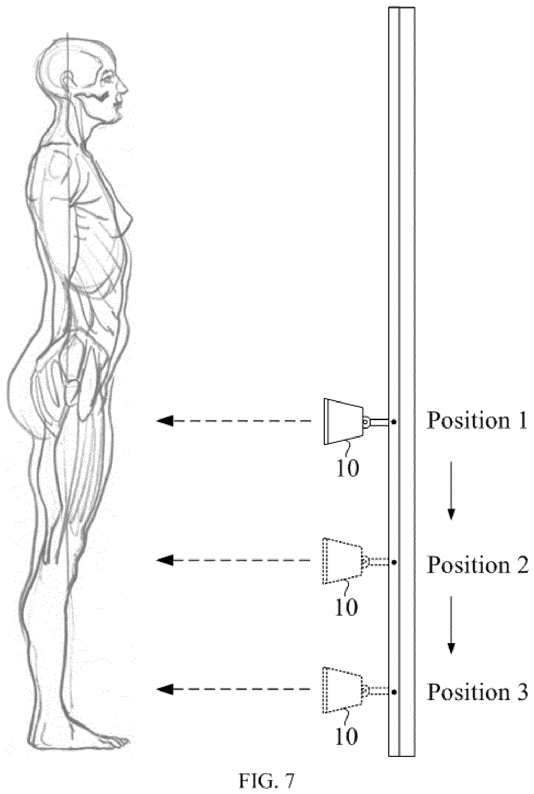

FIG. 7 shows an example of the stitching body part of an object under examination being lower limbs. Position 1, position 2 and position 3 shown in the figure are the first preset positions mentioned above. In this example, the X-ray source 10 is controlled to move to three positions (i.e. position 1, position 2 and position 3 shown in the figure) along an up-down direction (i.e. the direction of the vertical axis) and emit X-rays to the stitching body part at the three positions. It shall be noted that the dotted arrows in the figure are only used schematically to indicate the X-rays emitted by the X-ray source 10.

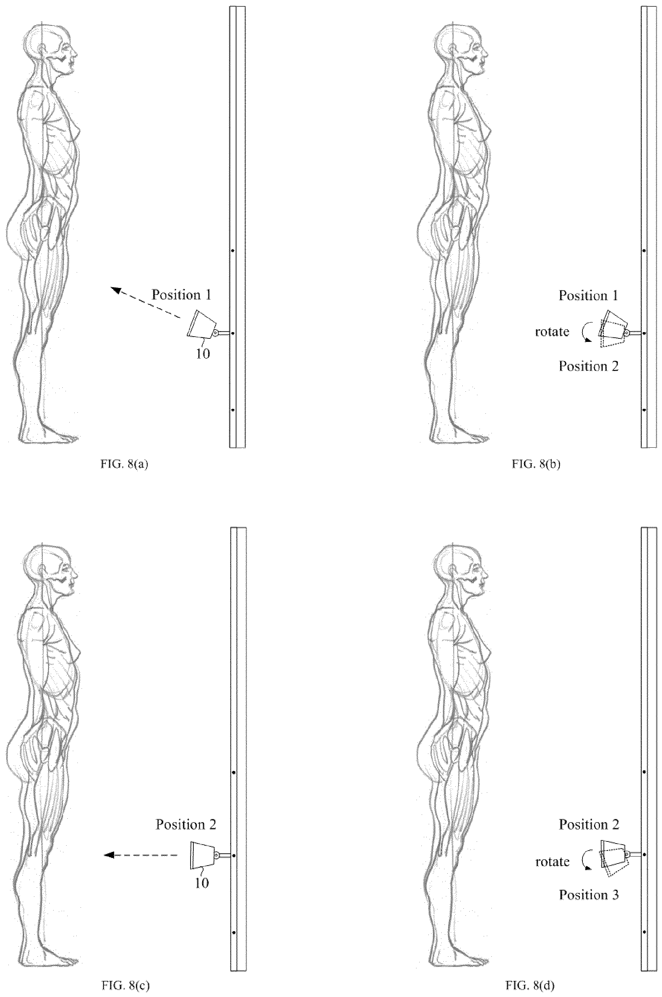

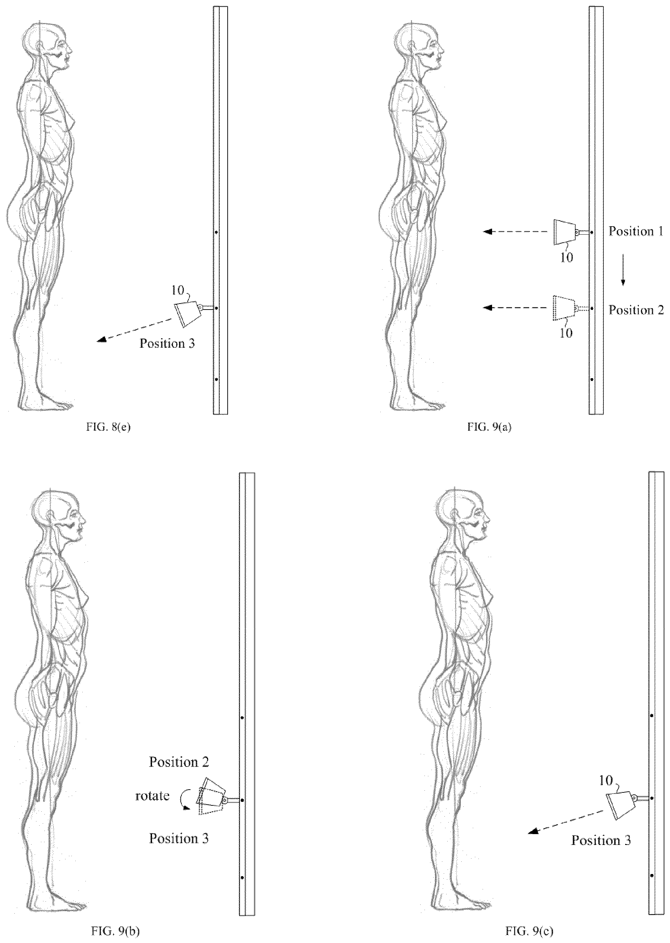

FIGs. 8(a) to 8(e) show an example of the stitching body part of an object under examination being lower limbs. Position 1, position 2 and position 3 shown in the figures are the first preset positions mentioned above. In this example, the X-ray source 10 is controlled to rotate/turn, instead of to displace, in the counterclockwise direction shown in the figure, to arrive at position 2 and position 3 in turn from position 1, and emit X-rays to the stitching body part at the three positions. FIG. 8(a) shows an example where the X-ray source 10 is at position 1 in the figure and emits X-rays to the stitching body part at position 1; FIG. 8(b) shows an example where the X-ray source 10 is rotated from position 1 (see the position of the X-ray source 10 drawn by the solid line in the figure) to position 2 (see the position of the X-ray source 10 drawn by the dotted line in the figure); FIG. 8(c) shows an example where the X-ray source 10 has been rotated to position 2 and emits X-rays at position 2 to the stitching body part; FIG. 8(d) shows an example where the X-ray source 10 is rotated from position 2 (see the position of the X-ray source 10 drawn by the solid line in the figure) to position 3 (see the position of the X-ray source 10 drawn by the dotted line in the figure); and FIG. 8(e) shows an example where the X-ray source 10 has been rotated to position 3 and emits X-rays at position 3 to the stitching body part. It shall be noted that the dotted arrows in the figures are only used schematically to indicate the X-rays emitted by the X-ray source 10.

FIGs. 9(a) to 9(e) show an example of the stitching body part of an object under examination being lower limbs. Position 1, position 2 and position 3 shown in the figures are the first preset positions mentioned above. In this example, during the whole process, the X-ray source 10 is controlled to displace firstly, and then turn/rotate shown in the counterclockwise direction in the figure; specifically, the X-ray source 10 is first displaced from position 1 to position 2 then rotated from position 2 to position 3, and emits X-rays to the stitching body part at these three positions. Referring to FIG. 9(a), the X-ray source 10 is at position 1 (see the position of the X-ray source 10 drawn by the solid line in the figure) and emits X-rays to the stitching body part at position 1; then, the X-ray source 10 is first moved from position 1 to position 2 (see the position of the X-ray source 10 drawn by the dotted line in the figure), and emits X-rays to the stitching body part at position 2. Next, as shown in FIG. 9(b), the X-ray source 10 is rotated from position 2 (the position of the X-ray source 10 drawn by the solid line in the figure) to position 3 (the position of the X-ray source 10 drawn by the dotted line in the figure). Then, as shown in FIG. 9 (c), after the X-ray source 10 has been rotated to position 3, it emits X-rays to the stitching body part at position 3. It shall be noted that the dotted arrows in the figure are only used schematically to indicate the X-rays emitted by the X-ray source 10.

It can be seen that the X-ray source being controlled to move to a plurality of first preset positions along one direction may be implemented by: only displacing the X-ray source 10, or only turning/rotating the X-ray source 10, or displacing and turning/rotating the X-ray source 10. This can be determined by an exposure parameter set of the stitching body part which may be configured in advance, or be configured or modified by user input.

The movement of the X-ray source 10 has been described above.

As mentioned above, the X-ray source 10 may be controlled to move to a plurality of first preset positions along one direction and emit X-rays to the stitching body part at each of the first preset positions; correspondingly, the detector 20 may need to receive the X-rays that have been emitted by the X-ray source at said each of the first preset positions and penetrating the stitching body part. This may be implemented in many ways, as explained in detail below.

In some examples, the detector 20 may be controlled to move to a plurality of second preset positions along one direction (such as the coronal axis direction of the object under examination), and when the detector 20 is moved to any of the second preset positions, the X-ray source 10 may be controlled to emit X-rays to the stitching body part and the detector 20 may be controlled to receive X-rays penetrating the stitching body part, thereby obtaining the plurality of digital X-ray images. The plurality of digital X-ray images may be used to be stitched to obtain the radiograph of the stitching body part.

In some examples, the digital X-ray imaging apparatus 01 may include a plurality of detectors 20; and during the process of the X-ray source 10 being moved to each of the plurality of first preset positions, the positions of the plurality of detectors 20 are kept stationary, and each of the plurality of detectors 20 is corresponded to each of the first preset positions at which the X-ray source 10 is moved and receives X-rays that have been emitted by the X-ray source 10 at said each of the first preset positions and penetrating the stitching body part, so that the plurality of digital X-ray images can be obtained. The plurality of digital X-ray images can be used to stitched to obtain the radiograph of the stitching body part.

In some examples, the size of the detector 20 is large enough, for example, larger than a first size; in this regard, during the process of the X-ray source 10 being moved to each of the plurality of first preset positions, the position of the detector 20 is kept stationary, and this detector 20 is used to receive X-rays that have been emitted by the X-ray source 10 at each of the first preset positions and penetrating the stitching body part, so that the plurality of digital X-ray images can be obtained. The plurality of digital X-ray images may be used to be stitched to obtain the radiograph of the stitching body part.

The above are some examples of how the detector 20 can receive X-rays emitted by the X-ray source 10 at each of the plurality of first preset positions and penetrating the stitching body part. It shall be understood that, other ways that the detector 20 can receive X-rays emitted by the X-ray source 10 at each of the plurality of first preset positions and penetrating the stitching body part are also in the protection scope of the present disclosure, which is not listed here.

The above describes some situations in which the X-ray source 10 and the detector 20 are controlled under the first stitching mode. The X-ray source 10 and the detector 20 may work together; accordingly, cooperation ways mentioned below can be obtained. It shall be noted that the following cooperation ways are just a few examples, and of course, there can be other cooperation ways, which will not be listed here.

In some examples, the X-ray source 10 is controlled to move to a plurality of first preset positions along one direction (such as the coronal axis direction of the object under examination) and the detector 20 is controlled to remain stationary relative to the X-ray source; and when the X-ray source 10 is moved to any of the first preset positions, the X-ray source 10 may be controlled to emit X-rays to the stitching body part and the detector 20 may be controlled to receive X-rays penetrating the stitching body part, thereby obtaining a plurality of digital X-ray images. The plurality of digital X-ray images may be used to stitched to obtain the radiograph of the stitching body part.

In some examples, the detector 20 is controlled to move to a plurality of second preset positions along one direction (such as the coronal axis direction of the object under examination) and the X-ray source 10 is controlled to remain stationary relative to the detector 20; and when the detector 20 is moved to any of the second preset positions, the X-ray source 10 may be controlled to emit X-rays to the stitching body part and the detector 20 may be controlled to receive X-rays penetrating the stitching body part, thereby obtaining a plurality of digital X-ray images. The plurality of digital X-ray images may be used to stitched to obtain the radiograph of the stitching body part.

In some examples, the X-ray source 10 is controlled to move to a plurality of first preset positions along one direction (such as the coronal axis direction of the object under examination) and the detector 20 is controlled to move to second preset positions corresponding to the plurality of first preset positions along one direction (such as the coronal axis direction of the object under examination); and when the X-ray source 10 and the detector 20 are both moved to any of their respective preset positions, the X-ray source 10 may be controlled to emit X-rays to the stitching body part and the detector 20 may be controlled to receive X-rays penetrating the stitching body part, thereby obtaining a plurality of digital X-ray images. The plurality of digital X-ray images may be used to stitched to obtain the radiograph of the stitching body part.

The above are some descriptions of the first stitching mode.

The following are some descriptions of the second stitching mode.

In some embodiments, the second stitching mode may include: obtaining a plurality of digital X-ray images while the X-ray source 10 and/or the detector 20 are/is controlled to move along at least two directions and the stitching body part is exposed, and stitching the plurality of digital X-ray images to obtain the radiograph of the stitching body part. The plurality of digital X-ray images are used to be stitched to obtain the radiograph of the stitching body part. The at least two directions are capable of determining a two-dimensional surface or a three-dimensional space. The two-dimensional surface may be a planar surface or a curved surface, for example, a cylindrical surface or an arc surface.

Accordingly, an imaging process may be as follows:
The processor 30 may determine a stitching body part of an object under examination, determine the digital imaging mode of the stitching body part, and when the digital imaging mode is the second stitching mode, control the X-ray source 10 and/or the detector 20 to move along at least two directions and expose the stitching body part to obtain a plurality of digital X-ray images which are then stitched to obtain the radiograph of the stitching body part. That is, the plurality of digital X-ray images are used to be stitched to get the radiograph of the stitching body part. The at least two directions may be capable of determining a two-dimensional surface or a three-dimensional space. The two-dimensional surface may be a planar surface or a curved surface, for example, a cylindrical surface or an arc surface.

In some embodiments, the obtaining of a plurality of digital X-ray images of the stitching body part by the processor 30 may include: under a current digital imaging mode, such as under the second stitching mode, obtaining the plurality of digital X-ray images while the X-ray source 10 and/or the detector 20 are/is controlled to move along at least two directions and the stitching body part is exposed. For example, a plurality of digital X-ray images may be obtained while the the X-ray source 10 and/or the detector 20 are/is controlled to move along at least two directions and the stitching body part is exposed; and the plurality of digital X-ray images may be obtained in real time by the processor 30.

The X-ray source 10 being controlled to move along at least two directions herein may refer to: automatically controlling the X-ray source 10 by the processor 30 to move along at least two directions, or manually controlling the X-ray source 10 by a user to move along at least two directions. The detector 20 being controlled to move along at least two directions herein may refer to: automatically controlling the detector 20 by the processor 30 to move along at least two directions, or manually controlling the detector 20 by a user to move along at least two directions.

In some examples, the X-ray source 10 is controlled to move to a plurality of first preset positions along at least two directions, and when the X-ray source 10 is moved to any of the first preset positions, the X-ray source is controlled to emit X-rays to the stitching body part. The plurality of first preset positions here may be characterized by multiple specific positions; in this regard, the X-ray source 10 is controlled to move to the multiple specific positions along the at least two directions and emit X-rays to the stitching body part at any of the multiple specific positions. Alternatively or additionally, the plurality of first preset positions may be characterized by a specific motion track; in this regard, the X-ray source 10 being controlled to move to a plurality of positions along at least two directions may refer to the X-ray source 10 being controlled to move along the motion track and emit X-rays to the stitching body part at any of the plurality of positions on the motion track.

The X-ray source 10 being controlled to move to a plurality of first preset positions along at least two directions herein may refer to: automatically controlling the X-ray source 10 by the processor 30 to move to a plurality of first preset positions along at least two directions, or manually controlling the X-ray source 10 by a user to move to plurality of first preset positions along at least two directions.

In some embodiments, the X-ray source 10 may be controlled to pass one part of the plurality of first preset positions along a first direction and emit X-rays to the stitching body part at the first preset positions passed, and the X-ray source 10 may be controlled to pass the other part of the plurality of first preset positions along a second direction and emit X-rays to the stitching body part at the first preset positions passed, wherein the first and second directions may form a two-dimensional surface. In some embodiments, the X-ray source 10 may be controlled to move along the first and second directions alternately to pass the plurality of first preset positions. The two-dimensional surface herein may be a planar surface or a curved surface which may be for example a cylindrical surface or an arc surface.

In some embodiments, the first and second directions are both straightline directions. In some embodiments, the first and second directions are both non straightline directions. In some embodiments, one of the first and second directions is a straightline direction (such as one of a coronal axis direction, a sagittal axis direction and a vertical axis direction of the object under examination), and the other is a non straightline direction.

In some embodiments, the non straightline directions may include at least one of a folded-line direction, a curve direction and a rotational direction. In some examples, the curve direction may be such as a direction of an arc.

In some embodiments, at least one of the first and second directions is a turning or rotational direction around a target center corresponding to the X-ray source 10. For example, the first and second directions are both a turning or rotational direction around a target center corresponding to the X-ray source 10. For another example, one of the first and second directions is a turning or rotational direction around a target center corresponding to the X-ray source 10, and the other is a straightline direction.

In some embodiments, the first direction is one of a coronal axis direction, a sagittal axis direction and a vertical axis direction of the object under examination, and the second direction is another of the coronal axis direction, the sagittal axis direction and the vertical axis direction of the object under examination.

The X-ray source 10 being controlled to pass one part of the plurality of first preset positions along the first direction here may refer to: automatically controlling the X-ray source 10 by the processor 30 to pass one part of the plurality of first preset positions along the first direction, or manually controlling the X-ray source 10 by a user to pass one part of the plurality of first preset positions along the first direction. The X-ray source 10 being controlled to pass the other part of the plurality of first preset positions along a second direction may refer to: automatically controlling the X-ray source 10 by the processor 30 to pass the other part of the plurality of first preset positions along the second direction, or manually controlling the X-ray source 10 by a user to pass the other part of the plurality of first preset positions along the second direction.

In some embodiments, the X-ray source 10 may be controlled to pass one part of the plurality of first preset positions along a first direction and emit X-rays to the stitching body part at the first preset positions passed, the X-ray source 10 may be controlled to pass another part of the plurality of first preset positions along a second direction and emit X-rays to the stitching body part at the first preset positions passed, and the X-ray source 10 may be controlled to pass yet another part (such as the remaining part) of the plurality of first preset positions along a third direction and emit X-rays to the stitching body part at the first preset positions passed, wherein the first, second and third directions form a three-dimensional space. It shall be understood that, said one part of the plurality of first preset positions, said another part of the plurality of first preset positions, and said the remaining part of the plurality of first preset positions may be three parts of the plurality of positions mentioned above, which form the plurality of positions mentioned above.

In some embodiments, the first, second and third directions are all straightline directions. In some embodiments, the first, second and third directions are all non straightline directions. In some embodiments, one of the first, second and third directions is a straightline direction or a non straightline direction.

In some embodiments, the non straightline direction may include at least one of a folded-line direction, a curve direction and a rotational direction. In some examples, the curve direction may be such as a direction of an arc.

In some embodiments, at least one of the first, second and third directions is a turning or rotational direction around a target center corresponding to the X-ray source 10. For example, the first, second and third directions are all a turning or rotational direction around a target center corresponding to the X-ray source 10. For another example, one of the first, second and third directions is a turning or rotational direction around a target center corresponding to the X-ray source 10, and the remaining two are both straightline directions, or a straightline direction and a folded-line direction. For yet another example, two of the first, second and third directions are a turning or rotational direction around a target center corresponding to the X-ray source 10, and the remaining one is a straightline direction or a folded-line direction.

In some embodiments, the first direction is one of a coronal axis direction, a sagittal axis direction and a vertical axis direction of the object under examination, and the second direction is another of a coronal axis direction, a sagittal axis direction and a vertical axis direction of the object under examination.

The X-ray source 10 being controlled to pass one part of the plurality of first preset positions along a first direction may refer to: automatically controlling the X-ray source 10 by the processor 30 to pass one part of the plurality of first preset positions along the first direction, or manually controlling the X-ray source 10 by a user to pass one part of the plurality of first preset positions along the first direction. The X-ray source 10 being controlled to pass another part of the plurality of first preset positions along a second direction may refer to: automatically controlling the X-ray source 10 by the processor 30 to pass another part of the plurality of first preset positions along the second direction, or manually controlling the X-ray source 10 by a user to pass another part of the plurality of first preset positions along the second direction. The X-ray source 10 being controlled to pass the remaining part of the plurality of first preset positions along a third direction may refer to: automatically controlling the X-ray source 10 by the processor 30 to pass the remaining part of the plurality of first preset positions along the third direction, or manually controlling the X-ray source 10 by a user to pass the remaining part of the plurality of first preset positions along the third direction.

The movement of the X-ray source 10 has been described above.

As mentioned above, the X-ray source 10 may emit X-rays to the stitching body part when it is moved to each of the plurality of first preset positions, correspondingly, the detector 20 may need to receive the X-rays emitted by the X-ray source 10 at said each first preset positions and penetrating the stitching body part. This may be implemented in many ways, as explained in detail below.

In some embodiments, the detector 20 may be controlled to move to a plurality of second preset positions along at least two directions, and when the detector 20 is moved to any of the second preset positions, the X-ray source 10 may be controlled to emit X-rays to the stitching body part and the detector 20 may be controlled to receive the X-rays penetrating the stitching body part, thereby obtaining the plurality of digital X-ray images which are used to be stitched to obtain the radiograph of the stitching body part.

In some examples, the detector 20 may be controlled to pass one part of the plurality of second preset positions along a first direction and receive the X-rays penetrating the stitching body part at the second preset positions passed, and the detector 20 may be controlled to pass the other part of the plurality of second preset positions along a second direction and receive the X-rays penetrating the stitching body part at the second preset positions passed, wherein the first and second directions form a two-dimensional surface. In some embodiments, the detector 20 may be controlled to move along the first and second directions alternately to pass the plurality of second preset positions. The two-dimensional surface herein may be a planar surface or a curved surface which may be for example a cylindrical surface or an arc surface.

In some embodiments, the first and second directions are both straightline directions. In some embodiments, the first and second directions are both non straightline directions. In some embodiments, one of the first and second directions is a straightline direction (such as one of a coronal axis direction, a sagittal axis direction and a vertical axis direction of the object under examination), and the other is a non straightline direction.

In some embodiments, the non straightline direction may include at least one of a folded-line direction, a curve direction and a rotational direction. In some examples, the curve direction may be such as a direction of an arc.

In some examples, the detector 20 may be controlled to pass one part of the plurality of second preset positions along a first direction and receive the X-rays penetrating the stitching body part at the second preset positions passed, the detector 20 may be controlled to pass another part of the plurality of second preset positions along a second direction and receive the X-rays penetrating the stitching body part at the second preset positions passed, and the detector 20 may be controlled to pass yet another part (such as the remaining part) of the plurality of second preset positions along a third direction and receive the X-rays penetrating the stitching body part at the second preset positions passed, wherein the first, second and third directions form a three-dimensional space.

In some embodiments, the first, second and third directions are all straightline directions. In some embodiments, the first, second and third directions are all non straightline directions. In some embodiments, one of the first, second and third directions is a straightline direction or a non straightline direction.

In some embodiments, the non straightline direction may include at least one of a folded-line direction, a curve direction and a rotational direction. In some examples, the curve direction may be such as a direction of an arc.

In some embodiments, the first direction is one of a coronal axis direction, a sagittal axis direction and a vertical axis direction of the object under examination, and the second direction is another of the coronal axis direction, the sagittal axis direction and the vertical axis direction of the object under examination.

In some examples, the digital X-ray imaging apparatus 01 may include a plurality of the detectors 20; in this regard, during the X-ray source 10 being moved to each of the plurality of first preset positions, the plurality of the detectors 20 are kept stationary, each of the detectors 20 may correspond to each of the plurality of first preset positions that the X-ray source 10 is moved and receive the X-rays that have been emitted by the X-ray source at said each first preset position and penetrating the stitching body part to obtain the plurality of digital X-ray images. The plurality of digital X-ray images may be used to be stitched to obtain the radiograph of the stitching body part.

In some examples, the size of the detector 20 is large enough, for example, larger than a first size; in this regard, during the process of the X-ray source 10 being moved to each of the plurality of first preset positions, the detector 20 is fixed in position and kept stationary, and this detector 20 is used to receive X-rays that have been emitted by the X-ray source 10 at each of the first preset positions and penetrating the stitching body part, so that the plurality of digital X-ray images can be obtained. The plurality of digital X-ray images may be used to be stitched to obtain the radiograph of the stitching body part.

In some examples, the size of the detector 20 is large enough, for example, larger than a second size and smaller than a first size; in this regard, the detector 20 has a size that allows it to fix in position and receive X-rays that have been emitted by the X-ray source 10 at a part of the plurality of first preset positions and penetrating the stitching body part, that is, when the X-ray source 10 is controlled to move to a part of the plurality of first preset positions and emit X-rays penetrating the stitching body part, the detector 20 which is fixed and unmoved is also capable of receiving X-rays that have been emitted by the X-ray source 10 at each of said part of the plurality of first preset positions and penetrating the stitching body part. It shall be noted that, the number of positions of said part is greater than one and smaller than a total number of the first preset positions. Taking the plurality of first preset positions as N first preset positions as an example, the detector 20 which only need to be located at M second preset positions may be capable of receiving the X-rays that have been emitted by the X-ray source 10 at each of the N first preset positions and penetrating the stitching body part, so as to obtain the plurality of digital X-ray images, where M is greater than 1 and less than N. The plurality of digital X-ray images are used to be stitched to obtain the radiograph of the stitching body part.

The above are some examples of how the detector 20 can receive X-rays that have been emitted by the X-ray source 10 at each of the plurality of first preset positions and penetrating the stitching body part. It shall be understood that, other ways that the detector 20 can receive X-rays that have been emitted by the X-ray source 10 at each of the plurality of first preset positions and penetrating the stitching body part are also in the protection scope of the present disclosure, which is not listed here.

Additionally, in an implementation of the detector 20 receiving X-rays that have been emitted by the X-ray source 10 at each of the plurality of first preset positions and penetrating the stitching body part; when it involves the movement of the detector 20, it may refer to: automatically controlling the detector 20 by the processor 30, or manually controlling the detector 20 by a user.

The above describes some situations in which the X-ray source 10 and the detector 20 are controlled under the second stitching mode. The X-ray source 10 and the detector 20 may work together; accordingly, cooperation ways thereof mentioned below can be obtained. It shall be noted that the following cooperation ways are just a few examples, and of course, there can be other cooperation ways, which will not be listed here.

In some examples, the X-ray source 10 is controlled to move to a plurality of first preset positions along at least two directions and the detector 20 is controlled to remain stationary, and when the X-ray source 10 is moved to any of the first preset positions, X-ray source 10 is controlled to emit X-rays to the stitching body part and the detector 20 is controlled to receive X-rays penetrating the stitching body part, thereby obtaining the plurality of digital X-ray images. The plurality of digital X-ray images are used to be stitched to obtain the radiograph of the stitching body part.

In some examples, the detector 20 may be controlled to move to a plurality of second preset positions along at least two directions and the X-ray source 10 is controlled to remain stationary, and when the detector is moved to any of the second preset positions, the X-ray source 10 is controlled to emit X-rays to the stitching body part and the the detector 20 is controlled to receive X-rays penetrating the stitching body part, so as to obtain the plurality of digital X-ray images. The plurality of digital X-ray images are used to be stitched to obtain the radiograph of the stitching body part.

In some examples, the X-ray source 10 is controlled to move to a plurality of first preset positions along at least two directions and the detector 20 may be controlled to move to second preset positions corresponding to the plurality of first preset positions along at least two directions, and when the X-ray source 10 and the detector 20 are both moved to any of their respective preset positions, the X-ray source 10 is controlled to emit X-rays to the stitching body part and the detector 20 is controlled to receive X-rays penetrating the stitching body part, thereby obtaining a plurality of digital X-ray images which may then be stitched to obtain a radiograph of the stitching body part.

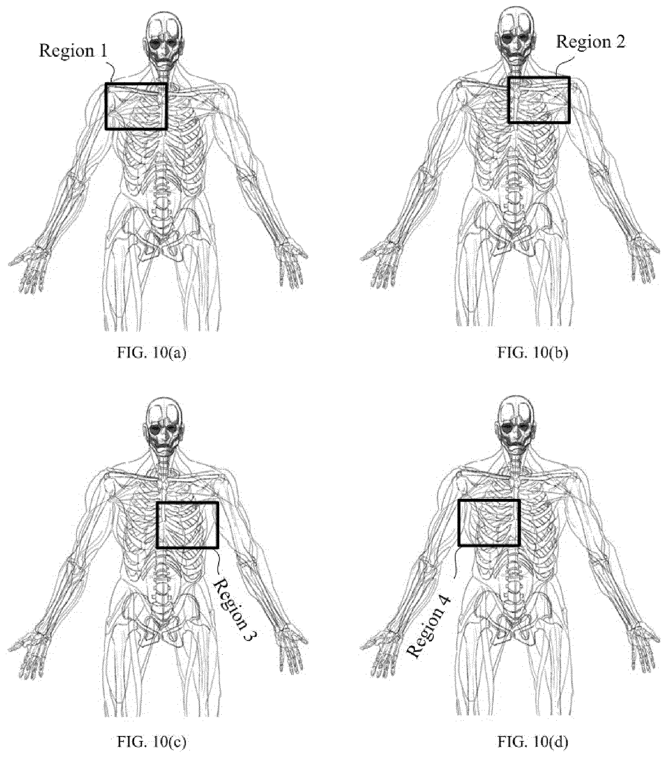

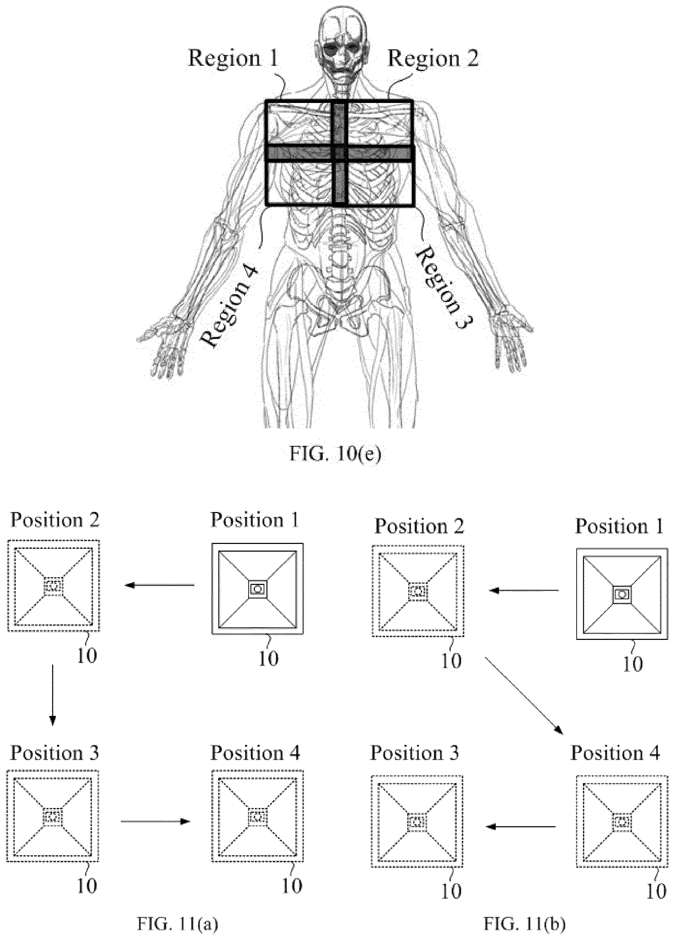

FIGs. 10(a) to 10(e) are an example of the stitching body part of an object under examination being a chest. As shown in the figures, the X-ray source 10 needs to emit X-rays respectively to the four regionsfour regions of the chest of the object under examination namely, region 1 (a region defined by the black solid line box in FIG. 10(a)), region 2 (a region defined by the black solid line box in FIG. 10(b)), region 3 (a region defined by the black solid line box in FIG. 10(c)) and region 4 (a region defined by the black solid line box in FIG. 10(d)), to obtain four digital X-ray images corresponding to the four regions, namely, a digital X-ray image 1, a digital X-ray image 2, a digital X-ray image 3 and a digital X-ray image 4, which are stitched to obtain the radiograph of the chest. FIG. 10(e) is a schematic diagram of the superposition of region 1, region 2, region 3 and region 4. It can be seen that there will be some overlapping regions between adjacent regions (see the regions filled with grayish black in the figure), which makes the digital X-ray images corresponding to adjacent regions also have some overlapping regions, that is, there will also be overlapping regions between adjacent images in digital X-ray image 1, digital X-ray image 2, digital X-ray image 3 and digital X-ray image 4. The overlapping regions between digital X-ray images may be called stitching regions. The existence of stitching regions between digital X-ray images can enable adjacent images to accurately match to complete image stitching.

FIG. 11(a) and FIG. 11(b) are two examples of performing digital X-ray imaging on the chest (i.e. the stitching body part of the object under examination) shown in FIG. 10 (e). Positions 1, 2, 3 and 4 in the figures are four first preset positions mentioned above. The directions of the plane of the paper in FIG. 11(a) and FIG. 11(b) are parallel to the coronal plane of the object under examination in FIG. 10(e), viewing from the perspective of the object under examination. In the example of FIG. 11(a), the X-ray source 10 is controlled to move from position 1 to position 2, position 3 and position 4 in turn, and emit X-rays to the stitching position at these four positions and the detector 20 is controlled to receive the X-rays, so as to obtain the digital X-ray image 1, the digital X-ray image 2, the digital X-ray image 3 and the digital X-ray image 4 respectively. In the example of FIG. 11(b), the X-ray source 10 is controlled to move from position 1 to position 2, position 4 and position 3 in turn, and emit X-rays to the stitching position at these four positions and the detector 20 is controlled to receive the X-rays, so as to obtain the digital X-ray image 1, the digital X-ray image 2, the digital X-ray image 4 and the digital X-ray image 3 respectively.

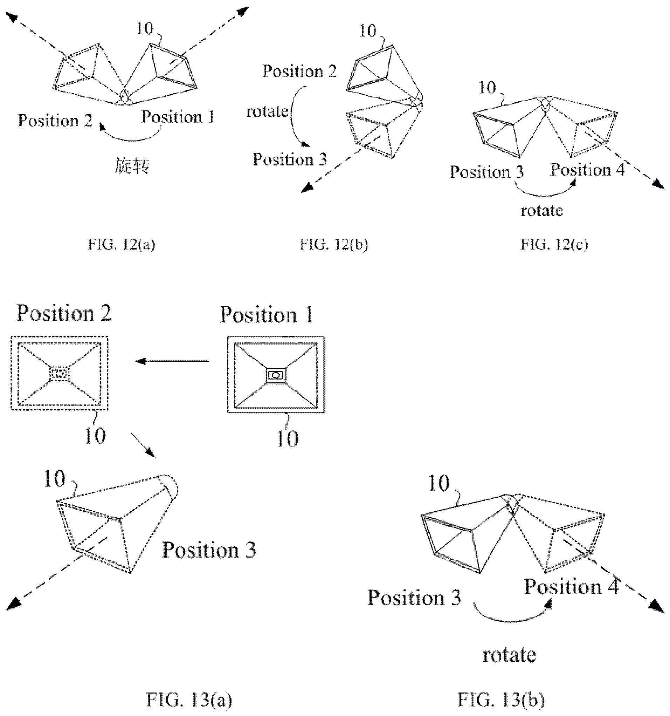

FIGs. 12 (a) to 12 (c) are an example of performing digital X-ray imaging on the chest (i.e. the stitching body part of the object under examination) shown in FIG. 10(e). Positions 1, 2, 3 and 4 in the figures are four first preset positions mentioned above. The directions of the plane of the paper in FIGs. 12(a) to 12(c) are parallel to the coronal plane of the object under examination in FIG. 10 (e), viewing from the perspective of the object under examination. In the example of FIGs. 12(a) to 12(c), the X-ray source 10, instead of being displaced, is turned/rotated. Please refer to FIG. 12(a), the X-ray source 10 is at position 1 (see the position of the X-ray source 10 drawn by the solid line in the figure) and emits X-rays to region 1 of the chest which are received by the detector 20 to obtain a digital X-ray image 1. Then the X-ray source 10 is rotated from position 1 (see the position of the X-ray source 10 drawn by the solid line in the figure) to position 2 (see the position of the X-ray source 10 drawn by the dotted line in the figure). After the X-ray source 10 has been rotated to position 2, the X-rays are emitted to region 2 of the chest at position 2 and are received by the detector 20 to obtain a digital X-ray image 2. Next, please refer to FIG. 12(b), the X-ray source 10 is rotated from position 2 (see the position of the X-ray source 10 drawn by the solid line in the figure) to position 3 (see the position of the X-ray source 10 drawn by the dotted line in the figure). After the X-ray source 10 has been rotated to position 3, the X-rays are emitted to the chest region 3 at position 3 and are received by the detector 20 to obtain the digital X-ray image 3. Then, please refer to FIG. 12(c), the X-ray source 10 is rotated from position 3 (see the position of the X-ray source 10 drawn by the solid line in the figure) to position 4 (see the position of the X-ray source 10 drawn by the dotted line in the figure). After the X-ray source 10 has been rotated to position 4, the X-rays are emitted to the chest region 4 at position 4 and are received by the detector 20 to obtain the digital X-ray image 4. It shall be noted that the dotted arrows in the figure are only used schematically to indicate the X-rays emitted by the X-ray source 10.

FIGs. 13(a) to 13(b) are an example of performing digital X-ray imaging on the chest (i.e. the stitching body part of the object under examination) shown in FIG. 10(e). Positions 1, 2, 3 and 4 in the figure are fourth first preset positions mentioned above. The direction of the plane of the paper in FIGs. 13(a) to 13(b) is parallel to the coronal plane of the object under examination in FIG. 10(e), viewing from the perspective of the object under examination. In the example of FIGs. 13(a) to 13(b), the X-ray source 10 moves first and then rotates/rotates during the whole process. Please refer to FIG. 13(a), X-rays are emitted by the X-ray source 10 to the chest region 1 at position 1 and are received by the detector 20 to obtain the digital X-ray image 1, the X-ray source 10 then moves from position 1 to position 2, and X-rays are emitted to the chest region 2 at position 2 and are received by the detector 20 to obtain the digital X-ray image 2. Then, the X-ray source 10 first moves from position 2 and then turns to position 3, and X-rays are emitted to the chest region 3 at position 3 and are received by the detector 20 to obtain the digital X-ray image 3. Next, please refer to FIG. 13(b), the X-ray source 10 is rotated from position 3 (see the position of the X-ray source 10 drawn by the solid line in the figure) to position 4 (see the position of the X-ray source 10 drawn by the dotted line in the figure). After the X-ray source 10 has been rotated to position 4, the X-rays are emitted to the chest region 4 at position 4 and are received by the detector 20 to obtain the digital X-ray image 4. It shall be noted that the dotted arrows in the figure are only used schematically to indicate the X-rays emitted by the X-ray source 10.

It can be seen that the X-ray source being controlled to move along at least two directions to a plurality of positions may be implemented by: only displacing the X-ray source 10, only turning/rotating the X-ray source 10, or displacing and turning/rotating the X-ray source 10. This can be determined by an exposure parameter set of the stitching body part which may be configured in advance, or be configured or modified by user input.

As mentioned above, the plurality of digital X-ray images are used to be stitched to obtain the radiograph of the stitching body part.

In some embodiments, the processor 30 may stitch the plurality of digital X-ray images in at least two stitching directions to obtain the radiograph of the stitching body part. It shall be understood that the stitching direction may refer to the direction of image synthesis in the process of image synthesis, which is the stitching of plurality of digital X-ray images to synthesize the radiograph of the stitching body part, which is not the same concept as the motion direction of the X-ray source 10 and the detector 20. Of course, in some examples, there may be some correlation between the stitching direction and the motion direction of the X-ray source 10 and/or the detector 20.

In some examples, the processor 30 may stitch at least two digital X-ray images from a plurality of digital X-ray images in a first stitching direction to obtain an intermediate stitching image, and the processor 30 may stitch the intermediate stitching image and at least one digital X-ray image from a plurality of digital X-ray images in a second stitching direction to obtain the radiograph of the stitching body part; wherein said at least two digital X-ray images are obtained while the X-ray source 10 and/or the detector 20 pass/passed a preset position along a first direction for exposure, and said at least one digital X-ray image is obtained while the X-ray source 10 and/or the detector 20 pass/passed a preset position along a second direction for exposure.

In some examples, the processor 30 may stitch at least two digital X-ray images from a plurality of digital X-ray images in the second stitching direction to obtain an intermediate stitching image, and the processor 30 may stitch the intermediate stitching image and at least one digital X-ray image from a plurality of digital X-ray images in the first stitching direction to obtain the radiograph of the stitching body part; wherein said at least two digital X-ray images may be obtained while the X-ray source 10 and/or the detector 20 pass/passes a preset position along the second direction for exposure, and said at least one digital X-ray image may be obtained while the X-ray source 10 and/or the detector 20 pass/passes a preset position along the first direction for exposure.

In some examples, the processor 30 may stitch at least three digital X-ray images from a plurality of digital X-ray images in the first stitching direction to obtain at least two intermediate stitching images, and the processor 30 may stitch the at least two intermediate stitching images in the second stitching direction to obtain the radiograph of the stitching body part; wherein the at least three digital X-ray images are obtained while the X-ray source 10 and/or the detector 20 pass/passes a preset position along the first direction for exposure.

In some examples, the processor 30 may stitch at least three digital X-ray images from a plurality of digital X-ray images in the second stitching direction to obtain at least two intermediate stitching images, and the processor 30 may stitch the at least two intermediate stitching images in the first stitching direction to obtain the radiograph of the stitching body part, wherein the at least three digital X-ray images are obtained while the X-ray source 10 and/or the detector 20 pass/passes a preset position along the second direction.

In some examples, the processor 30 may stitch, in the first stitching direction, at least two digital X-ray images obtained by being exposed along the first direction from the plurality of digital X-ray images to obtain an intermediate stitching image, and stitch, in the second stitching direction, the intermediate stitching image and at least one digital X-ray image obtained by being exposed along the second direction from the plurality of digital X-ray images to obtain the radiograph of the stitching body part.

Taking the digital X-ray images 1, 2, 3 and 4 corresponding to the regions 1, 2, 3 and 4 of the stitching body part obtained from performing exposing the stitching body part in FIG. 10(e) as an example to illustrate. Firstly, the digital X-ray image 1 and digital X-ray image 2 are stitched in a stitching direction to obtain a stitched image of the digital X-ray images 1 and 2 (it may be called the intermediate stitching image); then, the intermediate stitching image and the digital X-ray image 3 are stitched in another stitching direction, and the intermediate stitching image and the digital X-ray image 4 are also stitched in still another stitching direction (or it may be a stitched image of the digital X-ray images 1, 2 and 3, which is then stitched with the digital X-ray image 4), so as to obtain the radiograph of the stitching body part. It shall be noted that, the stitching may be performed in a counterclockwise direction, as well as in a clockwise direction, which is not limited here.

In some examples, the processor 30 may stitch, in the second stitching direction, at least two digital X-ray images obtained by being exposed along the second direction from the plurality of digital X-ray images to obtain an intermediate stitching image, and stitch, in the first stitching direction, the intermediate stitching image and at least one digital X-ray image obtained by being exposed along the first direction from the plurality of digital X-ray images to obtain the radiograph of the stitching body part.

Taking the digital X-ray images 1, 2, 3 and 4 corresponding to the regions 1, 2, 3 and 4 of the stitching body part obtained from performing exposing the stitching body part in FIG. 10(e) as an example to illustrate. Firstly, the digital X-ray images 1 and 4 are stitched in a stitching direction to obtain a stitched image of the digital X-ray images 1 and 4 (it may be called the intermediate stitching image); then, the intermediate stitching image and the digital X-ray image 2 are stitched in another stitching direction, and the intermediate stitching image and the digital X-ray image 3 are also stitched in still another stitching direction (or it may be a stitched image of the digital X-ray images 1, 4 and 2, which is then stitched with the digital X-ray image 3), so as to obtain the radiograph of the stitching body part. It shall be noted that, the stitching may be performed in a counterclockwise direction, as well as in a clockwise direction, which is not limited here.

In some examples, the processor 30 may stitch, in the first stitching direction, digital X-ray images obtained by being exposed along the first direction from the plurality of digital X-ray images to obtain at least two first intermediate stitching images, and stitch the at least two first intermediate stitching images to obtain the radiograph of the stitching body part.

Taking the digital X-ray images 1, 2, 3 and 4 corresponding to the regions 1, 2, 3 and 4 of the stitching body part obtained from performing exposing the stitching body part in FIG. 10(e) as an example to illustrate. Firstly, the digital X-ray images 1 and 2 are stitched in a stitching direction to obtain a stitched image of the digital X-ray images 1 and 2 (it may be called a first piece of the first intermediate stitching image); then, the digital X-ray images 4 and 3 are stitched in the same stitching direction (that is, a direction that is same to the stitching direction in which the digital X-ray images 1 and 2 are stitched) to obtain a stitched image of the digital X-ray images 4 and 3 (it may be called a second piece of the first intermediate stitching image); and then the first piece of the first intermediate stitching image and the second piece of the first intermediate stitching image are stitched in another stitching direction that is different from the one mentioned above to obtain the radiograph of the stitching body part.

In some examples, the processor 30 may stitch, in the second stitching direction, digital X-ray images obtained by being exposed along the second direction from the plurality of digital X-ray images to obtain at least two second intermediate stitching images, and stitch the at least two second intermediate stitching images to obtain the radiograph of the stitching body part.

Taking the digital X-ray images 1, 2, 3 and 4 corresponding to the regions 1, 2, 3 and 4 of the stitching body part obtained from performing exposing the stitching body part in FIG. 10(e) as an example to illustrate. Firstly, the digital X-ray images 1 and 4 are stitched in a stitching direction to obtain a stitched image of the digital X-ray images 1 and 4 (it may be called a first piece of the second intermediate stitching image); then, the digital X-ray images 2 and 3 are stitched in the same stitching direction (that is, a direction that is same to the stitching direction in which the digital X-ray images 1 and 4 are stitched) to obtain a stitched image of the digital X-ray images 2 and 3 (it may be called a second piece of the second intermediate stitching image); and then the first piece of the second intermediate stitching image and the second piece of the second intermediate stitching image are stitched in another stitching direction that is different from the one mentioned above to obtain the radiograph of the stitching body part.

It shall be understood that, after obtaining a plurality of digital X-ray images of the stitching body part, the plurality of digital X-ray images may be stitched to obtain the radiograph of the stitching body part in many ways. The above is just examples of several ways.

The above are some descriptions of the second stitching mode.

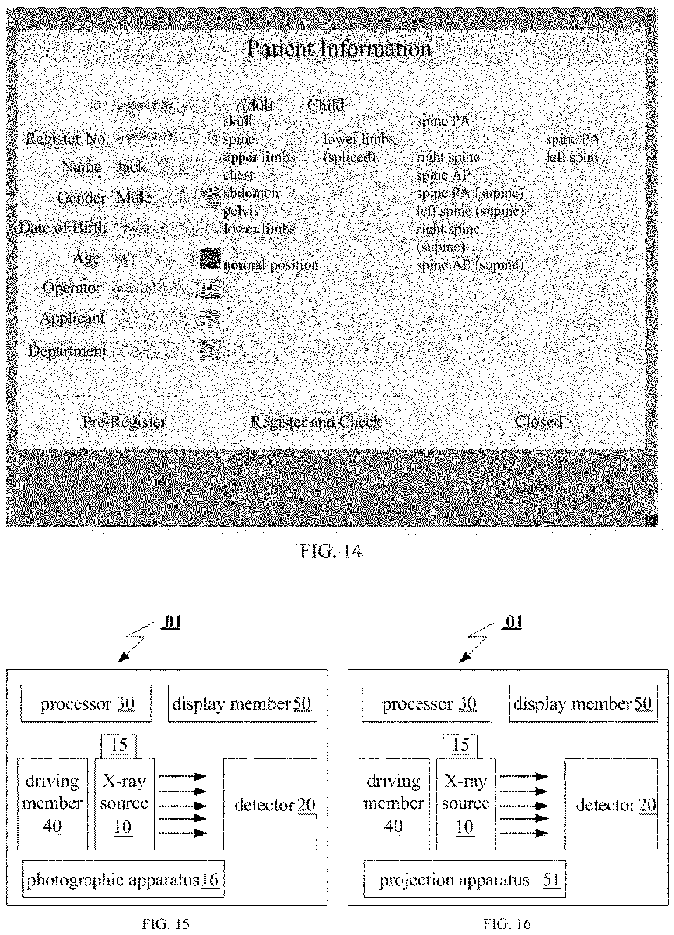

In some embodiments, the display member 50 may display options of exposable body part of the object under examination on a display interface, wherein the options of exposable body part may include at least one option of a stitching body part. The options of exposable body part may be ones presented in text or icon. FIG. 14 is such an example. The processor30 may determine the to-be-exposed body part of the object under examination from the options of exposable body part in response to a selection instruction on the options of the exposable body part. When the to-be-exposed body part is the stitching body part, the processor 30 may determine the exposure parameter set of the stitching body part. The exposure parameter set of the stitching body part may be automatically determined by the processor 30 according to the stitching body part of the object under examination, or be manually determined by a user via an input tool. In some embodiments, the exposure parameter set may include plurality of travel points (or positions) where the X-ray source 10 passes along at least two directions and the exposure parameters of the X-ray source 10 at the plurality of travel points, wherein the at least two directions are capable of determining a two-dimensional surface or a three-dimensional space. The two-dimensional surface herein may be a planar surface or a curved surface which may be for example a cylindrical surface or an arc surface. The processor 30 may, in response to an exposure instruction on the stitching body part, control the X-ray source 10 (for example driving the X-ray source 10 via the driving member 40) to pass the plurality of travel points along the at least two directions based on the exposure parameter set, and emit X-rays to the stitching body part at each of the travel points passed according to exposure parameters corresponding thereto. The processor 30 may control the detector 20 to receive the X-rays penetrating the stitching body part to obtain a plurality of digital X-ray images; and the processor 30 may stitch the plurality of digital X-ray images to obtain the radiograph of the stitching body part.

It shall be noted that, the plurality of travel points (or positions) involved herein may be characterized by multiple specific positions; in this regard, the X-ray source 10 may be controlled to pass the multiple specific positions along the at least two directions, and emit X-rays to the stitching body part at each of the specific positions passed according to the exposure parameters corresponding thereto. Additionally or alternatively, the plurality of travel points (or positions) may be characterized by a specific motion track; that is, the X-ray source 10 being controlled to pass the plurality of travel points along the at least two directions may mean that the X-ray source 10 may be controlled to move along the motion track, and emit X-rays to the stitching body part at multiple positions on the motion track, wherein each of the multiple positions corresponds to one of the travel points.

In some examples, the at least two directions are different. In addition, the directions represented by two parallel lines that are not on the same line are also different, so moving from one parallel line to the other must involve moving in a direction different from the parallel line.

In some embodiments, the processor 30 may, based on the exposure parameter set, control the X-ray source 10 to pass one part of the plurality of travel points along a first direction and emit X-rays to the stitching body part at each of the travel points passed according to exposure parameters corresponding thereto, and control the X-ray source 10 to pass the other part of the plurality of travel points along a second direction and emit X-rays to the stitching body part at each of the travel points passed according to exposure parameters corresponding thereto; wherein the first and second directions form a two-dimensional surface. In some embodiments, the processor30 may control the X-ray source 10 to move along the first and second directions alternately to pass the plurality of travel points based on the exposure parameter set. The two-dimensional surface herein may be a planar surface or a curved surface which may be for example a cylindrical surface or an arc surface.

In some embodiments, the first and second directions are both straightline directions. In some embodiments, the first and second directions are non straightline directions. In some embodiments, one of the first and second directions is a straightline direction (such as one of a coronal axis direction, a sagittal axis direction and a vertical axis direction of the object under examination), and the other is a non straightline direction.

In some embodiments, the non straightline direction may include at least one of a folded-line direction, a curve direction and a rotational direction. In some examples, the curve direction may be such as a direction of an arc.

In some embodiments, at least one of the first and second directions is a turning or rotational direction around a target center corresponding to the X-ray source 10. For example, the first and second directions are both a turning or rotational direction around a target center corresponding to the X-ray source 10. For another example, one of the first and second directions is a turning or rotational direction around a target center corresponding to the X-ray source 10, and the other is a straightline direction.

In some embodiments, the first direction is one of a coronal axis direction, a sagittal axis direction and a vertical axis direction of the object under examination, and the second direction is another of the coronal axis direction, the sagittal axis direction and the vertical axis direction of the object under examination.

In some embodiments, the processor 30 may, based on the exposure parameter set, control the X-ray source 10 to pass one part of the plurality of travel points along a first direction and emit X-rays to the stitching body part at each of the travel points passed according to exposure parameters corresponding thereto, control the X-ray source 10 to pass another part of the plurality of travel points along a second direction and emit X-rays to the stitching body part at each of the travel points passed according to exposure parameters corresponding thereto, and control the X-ray source 10 to pass yet another part (e.g. the remaining part) of the plurality of travel points along a third direction and emit X-rays to the stitching body part at each of the travel points passed according to exposure parameters corresponding thereto, wherein the first, second and third directions form a three-dimensional space. It shall be understood that, said one part of the plurality of travel points, said another part of the plurality of travel points and said yet another part (e.g. the remaining part) of the plurality of travel points are three parts of travel points in the plurality of travel points; and the three parts of travel points form the plurality of travel points.

In some embodiments, the first, second and third directions are all straightline directions. In some embodiments, the first, second and third directions are all non straightline directions. In some embodiments, one of the first, second and third directions is a straightline direction or a non straightline direction.

In some embodiments, the non straightline direction may include at least one of a folded-line direction, a curve direction and a rotational direction. In some examples, the curve direction may be such as a direction of an arc.

In some embodiments, at least one of the first, second and third directions is a turning or rotational direction around a target center corresponding to the X-ray source 10. For example, the first, second and third directions are all a turning or rotational direction around a target center corresponding to the X-ray source 10. For another example, one of the first, second and third directions is a turning or rotational direction around a target center corresponding to the X-ray source 10, and the remaining two are both straightline directions, or one is a straightline direction and the left is a folded-line direction. For yet another example, two of the first, second and third directions are a turning or rotational direction around a target center corresponding to the X-ray source 10, and the left is a straightline direction or a folded-line direction.

In some embodiments, the first direction is one of a coronal axis direction, a sagittal axis direction and a vertical axis direction of the object under examination, and the second direction is another of the coronal axis direction, the sagittal axis direction and the vertical axis direction of the object under examination.

The movement of the X-ray source 10 has been described above.

The following describes how the processor 30 controls the detector 20 to receive the X-ray penetrating the stitching body part to obtain the plurality of digital X-ray images.

In some examples, the processor 30 may control the detector 20 to move to a position corresponding to each travel point that the X-ray source 10 passes and where the X-ray source emits X-rays and receive X-rays emitted by the X-ray source at said each travel point and penetrating the stitching body part to obtain the plurality of digital X-ray images. For example, the processor 30 may control the X-ray source 10 and the detector 20 to displace synchronously or successively. In some example, the displacement of the detector may be in a straight line or a non-straight line such as a curve. For example, the detector may be moved along an arc. The position where the detector moves is aligned with the X-ray source, for example, the irradiation field corresponding to the X-ray source may be aligned with the center of the imaging area of the detector.

In some examples, the processor 30 may control the detector 20 to remain stationary relative to the X-ray source 10, and to receive X-rays emitted by the X-ray source at the travel points it passes and penetrating the stitching body part to obtain the plurality of digital X-ray images. For example, the processor 30 may control the X-ray source 10 and the detector 20 to displace synchronously. It shall be noted that, in some examples, the X-ray source 10 is moved in a turning or rotational direction around a target center corresponding to the X-ray source 10, where the target center may be such as the focus of the ball tube 13 or a nearby point. In this regard, if the detector 20 remains unmoved, the relative position relationship between it and the X-ray source has not fundamentally changed, so they are also relatively stationary.

In some examples, the size of the detector 20 is large enough, for example, larger than a first size; in this regard, during the process of the X-ray source 10 being moved to each of the plurality of travel points, the position of the detector 20 is kept stationary, and this detector 20 is used to receive X-rays that have been emitted by the X-ray source 10 at each of the travel points and penetrating the stitching body part, so that the plurality of digital X-ray images can be obtained.

The following describes how to determine the exposure parameter set of the stitching body part.

In some examples, the processor 30 may acquire a predetermined position relationship between the stitching body part and the detector 20, and determine the exposure parameter set of the stitching body part based on the predetermined position relationship. That is, a stitching body part is preset with a corresponding exposure parameter set, and the object under examination is guided by a user to carry out corresponding positioning; in this regard, the processor 30 may control the X-ray source 10 and the detector 20 to expose a current stitching body part of the object under examination according to a preset exposure parameter set corresponding to the current stitching body part.

In some examples, the processor 30 may acquire a current position relationship between the stitching body part of an object under examination and the detector 20 via a photographic apparatus 16, and determine the exposure parameter set of the stitching body part according to the current position relationship. In some examples, the photographic apparatus 16 can photograph to obtain a depth-sensing image containing three-dimensional spatial information involving the stitching body part and the detector 20; and the processor 30 may acquire the current position relationship between the stitching body part of the object under examination and the detector 20 through the depth-sensing image containing three-dimensional spatial information involving the stitching body part and the detector 20. In some examples, the photographic apparatus 16 can photograph to obtain an optical image containing two-dimensional spatial information involving the stitching body part; and the processor 30 may obtain a current position relationship between the stitching body part of the object under examination and the detector 20 base on the optical image containing two-dimensional spatial information involving the stitching body part, the current position of the detector 20 and a pre-established mapping relationship between the optical image and the spatial coordinates. Referring to FIG. 15, in the aforesaid example, the digital X-ray imaging apparatus 01 may include the photographic apparatus 16. In some examples, the display member 50 may display on a display interface or project by a projection apparatus 51 the current position relationship between the stitching body part and the detector 20; and the processor 30 may determine the exposure parameter set of the stitching body part based on a user operation. Referring to FIG. 16, in the aforesaid example, the digital X-ray imaging apparatus 01 may include the projection apparatus 51. It shall be understood that the projection apparatus 51 is a device for projecting images.

In some examples, the processor 30 may acquire at least one region of interest of the stitching body part, and determine travel points related to the at least one region of interest. The at least one region of interest may be outside the stitching region of the stitching body part, wherein the stitching region refers to a region formed between adjacent digital X-ray images from the plurality of digital X-ray images which are stitched to obtain the radiograph of the stitching body part.

In some examples, the exposure parameter set may include a plurality of travel points of the X-ray source 10 and exposure parameter(s) of the X-ray source 10 at the plurality of travel points; and the exposure parameter set may allow at least one region of interest to be totally exposed during digital X-ray imaging, as well as at least one region of interest being outside the stitching region. In other words, the processor 30 may determine the exposure parameter set of the stitching body part so that at least one region of interest is totally exposed during digital X-ray imaging and at least one region of interest is outside the stitching region, wherein the exposure parameter set includes a plurality of travel points of the X-ray source 10 and exposure parameter(s) of the X-ray source 10 at the plurality of travel points. In some embodiments, the processor 30 may, based on the at least one region of interest, acquire the exposure parameter set of the stitching body part.

The following describes the processor 30 acquiring at least one region of interest of the stitching body part.

In some embodiments, the processor 30 may control a display member 50 to display a first image of the stitching body part on a display interface; and in response to a user operation, the processor 30 may determine at least one region of interest of the stitching body part on the first image of the stitching body part.

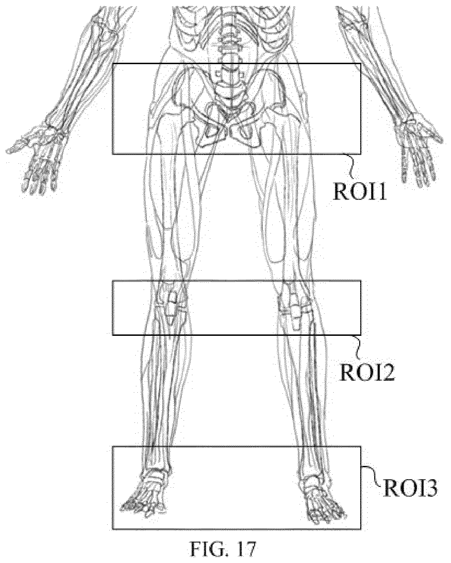

For example, please refer to the structure of the digital X-ray imaging apparatus 01 shown in FIG. 15. The digital X-ray imaging apparatus 01 may include a photographic apparatus 16 configured to perform imaging on at least the stitching body part to generate the above first image. In some embodiments, the first image may be displayed on the display member 50; and one or more regions of interest may be selected by a user via a mouse or a touch operation. FIG. 17 is an example showing lower limbs as the stitching part of the object under examination, on which three regions of interest, i.e. ROI 1, ROI 2 and ROI 3 in the figure, of the first image have been selected by a user.

It is intuitive and convenient for users to select the region(s) of interest on the image. The following describes how to determine the exposure parameter set of the stitching body part after the region(s) of interest have(has) been acquired.

In some embodiments, the processor 30 may, based on at least one region of interest determined on the first image, calculate a travel point and an X-ray exposure region that are associated with the region of interest, so that any region of interest can be completely covered by any individual X-ray exposure region which can also completely cover a stitching region used for stitching.

In some embodiments, the first image may include a depth-sensing image containing three-dimensional spatial information involving the stitching body part. In this regard, the photographic apparatus 16 is a depth-sensing camera. In some embodiments, the processor 30 may, based on at least one region of interest determined on the depth-sensing image, obtain spatial coordinates corresponding to the region of interest and may, based on the spatial coordinates, calculate a travel point and an X-ray exposure region that are associated with the region of interest, so that any region of interest can be completely covered by any individual X-ray exposure region which can also cover the stitching region used for stitching.

In some embodiments, the first image may include an optical image containing two-dimensional spatial information involving the stitching body part. In some embodiments, the processor 30 may, based on at least one region of interest determined on the optical image and a pre-established mapping relationship between the optical image and spatial coordinates, obtain spatial coordinates corresponding to the region of interest, and may calculate a travel point and an X-ray exposure region that are associated with the region of interest, so that any region of interest can be completely covered by any individual X-ray exposure region which can also cover the stitching region used for stitching.

The processor 30 may, at least based on a travel point and an X-ray exposure region that are associated with the region of interest, acquire an exposure parameter set. In some embodiments, the processor 30 may, based on a travel point and an X-ray exposure region that are associated with a region of interest, determine whether the stitching body part has a gap region that is uncovered by the X-ray exposure region associated with the region of interest. When there has the gap region, the processor 30 may acquire the gap region and acquire a travel point and an X-ray exposure region that are associated with the gap region, so that the gap region is completely covered without overlapping with any region of interest; and the processor 30 may, based on the travel point and the X-ray exposure region that are associated with the region of interest, as well as the travel point and the X-ray exposure region that are associated with the gap region, acquire an exposure parameter set. When there has no gap region, the processor 30 may, based on the travel point and the X-ray exposure region that are associated with the region of interest, acquire an exposure parameter set. For example,

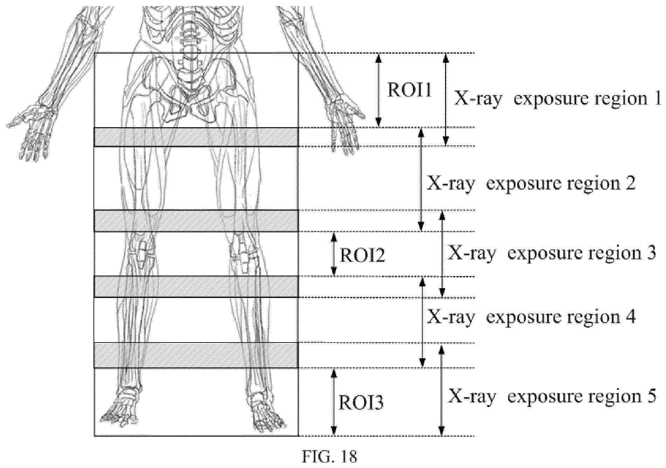

FIG. 18 is a schematic diagram of obtaining the exposure parameter set based on the region of interest selected as shown in FIG. 17. The gray shaded region in the figure is the stitching region. It can be seen that the regions of interest ROI 1, ROI 2 and ROI 3 are completely covered by any individual X-ray exposure region which also completely covers the stitching region used used for stitching. The regions of interest ROI 1, ROI 2 and ROI 3 are not in the stitching region.

The above is based on the description of acquiring the region of interest on the first image and further acquiring the exposure parameter set.

The following describes how to calibrate the region of interest by means of an X-ray beam limiter 15.

In some embodiments, when receiving a triggering instruction to calibrate a region of interest, the processor 30 may acquire a travel point of the X-ray source 10 and an irradiation field of the X-ray beam limiter 15 under the triggering instruction to calibrate at least one region of interest of the stitching body part. In some embodiments, the processor 30 may take a region corresponding to the irradiation field of the X-ray beam limiter 15 as the region of interest. In some embodiments, the processor 30 may, according to the irradiation field of the X-ray beam limiter 15 under the triggering instruction, determine a travel point of interest of the stitching body part, and determine a region of interest based on the travel point of interest; for example, generating a region with a preset size centered on the travel point of interest and taking the region with a preset size as the region of interest. In some embodiments, the length of the region with a preset size in the first direction (e.g. the coronal axis direction or the sagittal axis direction) is less than a first threshold. In some embodiments, the length of the region with a preset size in the second direction (e.g. the vertical axis direction) is less than a second threshold. In some embodiments, the first threshold is 27cm to 25cm. In some embodiments, the second threshold is 9cm to 7cm.

The X-ray source 10 may be displaced by a user to drive the X-ray beam limiter to displace together, and when the X-ray beam limiter 15 irradiates a region of interest, the region of interest may be calibrated by sending the triggering instruction to the processor 30 via the digital X-ray imaging apparatus 01 by a user.

The following describes how to determine the exposure parameter set of the stitching body part after acquiring the region of interest.

In some embodiments, the processor 30 may take the travel point of the X-ray source 10 under the triggering instruction as the travel point associated with the region of interest, and take the irradiation field of the X-ray beam limiter 15 under the triggering instruction as the X-ray exposure region associated with the region of interest; and the processor 30 may acquire the exposure parameter set at least according to the travel point and the X-ray exposure region that are associated with the region of interest. In some embodiments, the processor 30 may, according to the travel point and the X-ray exposure region that are associated with the region of interest, determine whether the stitching body part has a gap region that is uncovered by the X-ray exposure region associated with the region of interest. When there has the gap region, the processor 30 may acquire the gap region and acquire a travel point and an X-ray exposure region that are associated with the gap region, so that the gap region is completely covered without overlapping with any region of interest; and the processor 30 may, based on the travel point and the X-ray exposure region that are associated with the region of interest, as well as the travel point and the X-ray exposure region that are associated with the gap region, acquire an exposure parameter set. When there has no gap region, the processor 30 may, based on the travel point and the X-ray exposure region that are associated with the region of interest, acquire an exposure parameter set.

The above is the description of how to calibrate the region of interest through the irradiation field of the X-ray beam limiter 15 and further to acquire the exposure parameter set.

It can be seen that in some embodiments, the exposure parameters in the exposure parameter set may at least include the X-ray irradiation region.

In some embodiments, the processor 30 may determine the stitching body part of the object under examination. For example, the display member 50 may display options of exposable body part of the object under examination on a display interface. The options of exposable body part may include at least one option of a stitching body part. The options of exposable body part may be ones presented in text or icon. In response to a selection instruction on the options of the exposable body part, the processor 30 may determine the to-be-exposed body part of the object under examination from the options of exposable body part. The processor 30 may obtain a plurality of digital X-ray images of the stitching body part, wherein the plurality of digital X-ray images may be obtained while the X-ray source 10 and/or the detector 20 are/is control to move along at least one non straightline direction and the stitching body part is exposed. The processor 30 may stitch the plurality of digital X-ray images to obtain the radiograph of the stitching body part.

In some embodiments, the processor 30 obtaining the plurality of digital X-ray images of the stitching body part may include: obtaining the plurality of digital X-ray images based on historical images. The historical images here may refer to digital X-ray images that have been exposed and stored in advance.

In some embodiments, the processor 30 obtaining the plurality of digital X-ray images of the stitching body part may include: under a current digital imaging mode, obtaining the plurality of digital X-ray images while the X-ray source 10 and/or the detector 20 are/is controlled to move along at least a non straightline direction and the the stitching body part is exposed.

In some embodiments, the X-ray source 10 may be controlled to move to a plurality of first preset positions along at least a non straightline direction and the detector 20 is controlled to remain stationary, and when the X-ray source 10 is moved to any of the first preset positions, the X-ray source 10 is controlled to emit X-rays to the stitching body part and the detector 20 is controlled to receive X-rays penetrating the stitching body part, so as to obtain the plurality of digital X-ray images.

In some embodiments, the detector 20 may be controlled to move to a plurality of second preset positions along at least a non straightline direction and the X-ray source 10 is controlled to remain stationary, and when the detector 20 is moved to any of the second preset positions, the X-ray source 10 is controlled to emit X-rays to the stitching body part and the the detector 20 is controlled to receive the X-rays penetrating the stitching body part, so as to obtain the plurality of digital X-ray images.

In some embodiments, the X-ray source 10 is controlled to move to a plurality of first preset positions along at least a non straightline direction and the detector 20 may be controlled to move to second preset positions corresponding to the plurality of first preset positions along at least a non straightline direction; and when the X-ray source 10 and the detector 20 are both moved to any of their respective preset positions, the X-ray source 10 is controlled to emit X-rays to the stitching body part and the detector 20 is controlled to receive X-rays penetrating the stitching body part, so as to obtain the plurality of digital X-ray images.

In some embodiments, the non straightline direction may include at least one of a folded-line direction, a curve direction and a rotational direction.

The above are some explanations of the digital X-ray imaging apparatus 01 in the present disclosure.

A digital X-ray imaging method is also disclosed in some embodiments of the present disclosure; and the digital X-ray imaging method in some embodiments may be applied to the digital X-ray imaging apparatus 01 disclosed in some embodiments of present disclosure.

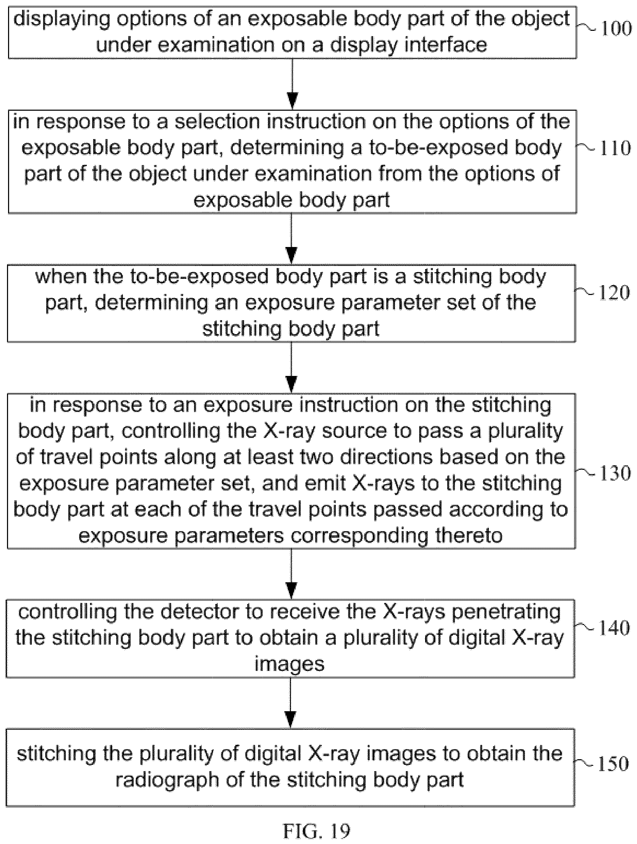

Referring to FIG. 19, the digital X-ray imaging method in some embodiments may include steps as follows:
Step 100: displaying options of exposable body part of the object under examination on a display interface, the options of exposable body part comprising at least an option of a stitching body part;
Step 110: in response to a selection instruction on the options of the exposable body part, determining a to-be-exposed body part of the object under examination from the options of exposable body part; and
Step 120: when the to-be-exposed body part is a stitching body part, determining an exposure parameter set of the stitching body part. In some embodiments, the exposure parameter set may include a plurality of travel points that the X-ray source 10 is capable of passing along at least two directions and exposure parameters about the X-ray source 10 at the plurality of travel points.

In some examples, the at least two directions may be capable of determining a two-dimensional surface or a three-dimensional space. The two-dimensional surface herein may be a planar surface or a curved surface which may be for example a cylindrical surface or an arc surface.

There are many ways to determine the exposure parameter set of the stitching body part in step 120.

In some examples, in step 120, a predetermined position relationship between the stitching body part and the detector 20 may be obtained, and the exposure parameter set of the stitching body part may be determined based on the predetermined position relationship. That is, a stitching body part is preset with a corresponding exposure parameter set, and the object under examination is guided by a user to carry out corresponding positioning; in this regard, the processor 30 may control the X-ray source 10 and the detector 20 to expose a current stitching body part of the object under examination according to a preset exposure parameter set corresponding to the current stitching body part.

In some examples, in step 120, a current position relationship between the stitching body part of the object under examination and the detector 20 may be obtained by a photographic apparatus 16, and an exposure parameter set of the stitching body part may be determined according to the current position relationship. In some examples, the photographic apparatus 16 can photograph to obtain a depth-sensing image containing three-dimensional spatial information involving the stitching body part and the detector 20. In step 120, the current position relationship between the stitching body part of the object under examination and the detector 20 may be obtained by a depth-sensing image containing three-dimensional spatial information involving the stitching body part and the detector 20. In some examples, the photographic apparatus 16 can photograph to obtain an optical image containing two-dimensional spatial information involving the stitching body part; and in step 120, based on the optical image containing two-dimensional spatial information involving the stitching body part, a current position of the detector 20 and a pre-established mapping relationship between the optical image and the spatial coordinates, the current position relationship between the stitching body part of the object under examination and the detector 20 may be obtained. Referring to FIG. 15, in the aforesaid example, the digital X-ray imaging apparatus 01 may include the photographic apparatus 16. In some examples, the display member 50 may display on a display interface or project by a projection apparatus 51 the current position relationship between the stitching body part and the detector 20; and the processor 30 may determine the exposure parameter set of the stitching body part based on a user operation. Referring to FIG. 16, in the aforesaid example, the digital X-ray imaging apparatus 01 may include the projection apparatus 51. It shall be understood that the projection apparatus 51 is a device for projecting images.

In some examples, in step 120, at least one region of interest of the stitching body part may be acquired, and travel points related to the at least one region of interest may be determined. The at least one region of interest may be outside the stitching region of the stitching body part, wherein the stitching region refers to a region formed between adjacent digital X-ray images from the plurality of digital X-ray images which are stitched to obtain the radiograph of the stitching body part.

Step 130: in response to an exposure instruction on the stitching body part, controlling the X-ray source 10 to pass a plurality of travel points along at least two directions based on the exposure parameter set, and emit X-rays to the stitching body part at each of the travel points passed according to exposure parameters corresponding thereto.

For example, in step 130, the X-ray source 10 may be controlled to pass one part of the plurality of travel points along a first direction based on the exposure parameter set and emit X-rays to the stitching body part at each of the travel points passed according to exposure parameters corresponding thereto, and the X-ray source 10 may be controlled to pass the other part of the plurality of travel points along a second direction and emit X-rays to the stitching body part at each of the travel points passed according to exposure parameters corresponding thereto, wherein the first and second directions may form a two-dimensional surface. In some embodiments, the processor30 may control the X-ray source 10 to move along the first and second directions alternately to pass the plurality of travel points based on the exposure parameter set. The two-dimensional surface herein may be a planar surface or a curved surface which may be for example a cylindrical surface or an arc surface.

In some embodiments, the first and second directions are both straightline directions. In some embodiments, the first and second directions are non straightline directions. In some embodiments, one of the first and second directions is a straightline direction (such as one of a coronal axis direction, a sagittal axis direction and a vertical axis direction of the object under examination), and the other is a non straightline direction.

In some embodiments, the non straightline direction may include at least one of a folded-line direction, a curve direction and a rotational direction. In some examples, the curve direction may be such as a direction of an arc.

In some embodiments, the first direction is one of a coronal axis direction, a sagittal axis direction and a vertical axis direction of the object under examination, and the second direction is another of the coronal axis direction, the sagittal axis direction and the vertical axis direction of the object under examination.

For example, in step 130, the X-ray source 10 may be controlled to pass one part of the plurality of travel points along a first direction based on the exposure parameter set and emit X-rays to the stitching body part at each of the travel points passed according to exposure parameters corresponding thereto, the X-ray source 10 may be controlled to pass another part of the plurality of travel points along a second direction and emit X-rays to the stitching body part at each of the travel points passed according to exposure parameters corresponding thereto, and the X-ray source 10 may be controlled to pass yet another part (e.g. the remaining part) of the plurality of travel points along a third direction and emit X-rays to the stitching body part at each of the travel points passed according to exposure parameters corresponding thereto; wherein the first, second and third directions may form a three-dimensional space. It shall be understood that, said one part of the plurality of travel points, said another part of the plurality of travel points and said yet another part (e.g. the remaining part) of the plurality of travel points are three parts of travel points in the plurality of travel points; and the three parts of travel points form the plurality of travel points.

In some embodiments, the first, second and third directions are all straightline directions. In some embodiments, the first, second and third directions are all non straightline directions. In some embodiments, one of the first, second and third directions is a straightline direction or a non straightline direction.

In some embodiments, the non straightline direction may include at least one of a folded-line direction, a curve direction and a rotational direction. In some examples, the curve direction may be such as a direction of an arc.

In some embodiments, the first direction is one of a coronal axis direction, a sagittal axis direction and a vertical axis direction of the object under examination, and the second direction is another of the coronal axis direction, the sagittal axis direction and the vertical axis direction of the object under examination.

Step 140: controlling the detector 20 to receive the X-rays penetrating the stitching body part to obtain a plurality of digital X-ray images.

For example, in step 140, the the detector 20 may be controlled to move to a position corresponding to each travel point that the X-ray source 10 passes and where the X-ray source emits X-rays and receive X-rays emitted by the X-ray source at said each travel point and penetrating the stitching body part to obtain the plurality of digital X-ray images.

For another example, the detector 20 may be controlled to remain stationary relative to the X-ray source 10, and to receive X-rays emitted by the X-ray source at the travel points it passes and penetrating the stitching body part to obtain the plurality of digital X-ray images.

Step 150: stitching the plurality of digital X-ray images to obtain the radiograph of the stitching body part.

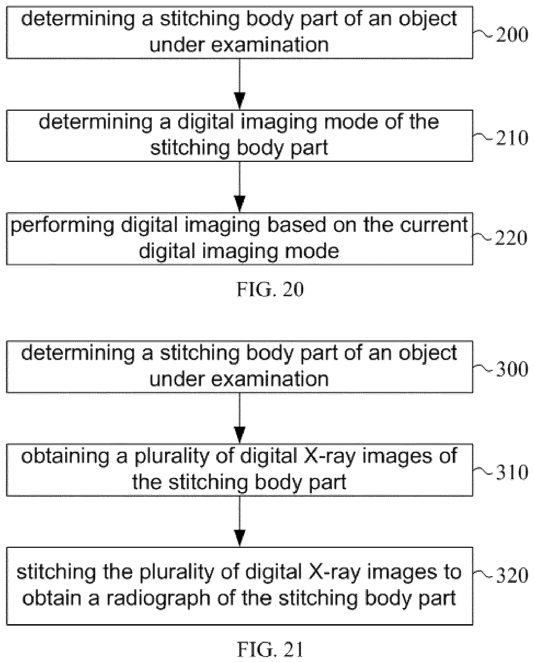

Referring to FIG. 20, a digital X-ray imaging method in some embodiments may include steps as follows:
Step 200: determining a stitching body part of an object under examination;
Step 210: determining a digital imaging mode of the stitching body part; and
For example, in step 210, options of digital imaging mode may be displayed on a display interface. In step 210, the digital imaging mode may be determined in response to a selection instruction on the options of digital imaging mode.

For another example, in step 210, the digital imaging mode may automatically determined according to the stitching body part.

In some embodiments, the digital imaging mode may include a first stitching mode and a second stitching mode; wherein the first stitching mode may include obtaining a plurality of digital X-ray images while the X-ray source 10 and/or the detector 20 are/is controlled to move along a direction and the stitching body part is exposed, and stitching the plurality of digital X-ray images to obtain a radiograph of the stitching body part, and the second stitching mode may include obtaining a plurality of digital X-ray images while the X-ray source 10 and/or the detector 20 are/is controlled to move along at least two directions and the stitching body part is exposed, and stitching the plurality of digital X-ray images to obtain a radiograph of the stitching body part. Said at least two directions may be capable of determining a two-dimensional surface or a three-dimensional space. The two-dimensional surface herein may be a planar surface or a curved surface which may be for example a cylindrical surface or an arc surface.

In some examples, in step 210, digital imaging modes may be switched among in response to a triggering of a mode switching button.

Step 220: performing digital imaging based on the current digital imaging mode.

In some embodiments, in step 220, when the current digital imaging mode is the first stitching mode, a plurality of digital X-ray images may be obtained while the X-ray source 10 and/or the detector 20 are/is controlled to move along a direction and the stitching body part is exposed, and the plurality of digital X-ray images may be stitched to obtain a radiograph of the stitching body part.

In some examples, in step 220, the X-ray source 10 may be controlled to move to a plurality of first preset positions along one direction and the detector 20 may be controlled to remain stationary, and when the X-ray source 10 is moved to the first preset positions, the X-ray source 10 may be controlled to emit X-rays to the stitching body part and the detector 20 may be controlled to receive the X-rays penetrating the stitching body part to obtain the plurality of digital X-ray images, and the plurality of digital X-ray images may be stitched to obtain a radiograph of the stitching body part.

In some examples, in step 220, the detector 20 may be controlled to move to a plurality of second preset positions along a direction and the X-ray source 10 may be controlled to remain stationary, and when the detector 20 is moved to any of the second preset positions, the X-ray source 10 may be controlled to emit X-rays to the the stitching body part and the detector 20 may be controlled to receive the X-rays penetrating the stitching body part to obtain the plurality of digital X-ray images, and the plurality of digital X-ray images may be stitched to obtain a radiograph of the stitching body part.

In some examples, in step 220, the X-ray source 10 may be controlled to move to a plurality of first preset positions along a direction and the detector 20 may be controlled to move to second preset positions corresponding to the plurality of first preset positions along a direction, and when the X-ray source 10 and the detector 20 are both moved to any of their respective preset positions, the X-ray source 10 is controlled to emit X-rays to the stitching body part and the detector 20 is controlled to receive X-rays penetrating the stitching body part to obtain the plurality of digital X-ray images, and the plurality of digital X-ray images may be stitched to obtain a radiograph of the stitching body part.

In some examples, in step 220, when the digital imaging mode is the second stitching mode, the plurality of digital X-ray images may be obtained while the X-ray source 10 and/or the detector 20 are/is controlled to move along at least two directions and the stitching body part is exposed, and the plurality of digital X-ray images may be stitched to obtain a radiograph of the stitching body part.

In some examples, in step 220, the X-ray source 10 may be controlled to move to a plurality of first preset positions along at least two directions and the detector 20 may be controlled to remain stationary; and when the X-ray source 10 is moved to the first preset positions, the X-ray source 10 may be controlled to emit X-rays to the stitching body part and the detector 20 may be controlled to receive the X-rays penetrating the stitching body part to obtain the plurality of digital X-ray images, and the plurality of digital X-ray images may be stitched to obtain a radiograph of the stitching body part.

In some examples, in step 220, the detector 20 may be controlled to move to a plurality of second preset positions along at least two directions and the X-ray source 10 may be controlled to remain stationary; and when the detector 20 is moved to the second preset positions, the X-ray source 10 may be controlled to emit X-rays to the stitching body part and the detector 20 may be controlled to receive X-rays penetrating the stitching body part to obtain the plurality of digital X-ray images, and the plurality of digital X-ray images may be stitched to obtain a radiograph of the stitching body part.

In some examples, in step 220, the X-ray source 10 may be controlled to move to a plurality of first preset positions along at least two directions and the detector 20 may be controlled to move to second preset positions corresponding to the plurality of first preset positions along at least two directions; and when the X-ray source 10 and the detector 20 are both moved to any of their respective preset positions, the X-ray source 10 may be controlled to emit X-rays to the stitching body part and the detector 20may be controlled to receive X-rays penetrating the stitching body part to obtain the plurality of digital X-ray images, and the plurality of digital X-ray images may be stitched to obtain a radiograph of the stitching body part.

In some examples, the X-ray source 10 being controlled to move to a plurality of first preset positions along at least two directions in step 220 may include: controlling the X-ray source 10 to pass one part of the plurality of first preset positions along a first direction and emit X-rays to the stitching body part at any of the first preset positions passed, controlling the X-ray source 10 to pass the other part of the plurality of first preset positions along a second direction to emit X-rays to the stitching body part at any of the first preset positions passed, wherein the first and second directions may form a two-dimensional surface, and the at least two directions may include the first and second directions. The first and second directions are both straightline directions; or, the first and second directions are non straightline directions; or, one of the first and second directions is a straightline direction and the other is a non straightline direction.

Alternatively or additionally, in some examples, the X-ray source 10 being controlled to move to a plurality of first preset positions along at least two directions may include: controlling the X-ray source 10 to pass one part of the plurality of first preset positions along a first direction and emit X-rays to the stitching body part at any of the first preset positions passed, controlling the X-ray source 10 to pass another part of the plurality of first preset positions along a second direction and emit X-rays to the stitching body part at any of the first preset positions passed, and controlling the X-ray source 10 to pass yet another part (e.g. the remaining part) of the plurality of first preset positions along a third direction and emit X-rays to the stitching body part at any of the first preset positions passed, wherein the first, second and third directions may form a three-dimensional space, and the at least two directions may include the first, second and third directions. The first, second and third directions are all straightline directions; or, the first, second and third directions are non straightline directions; or, one of the first, second and third directions is a straightline direction or a non straightline direction.

In some examples, in step 220, the X-ray source 10 may be controlled to move long the first and second directions alternately to pass the plurality of first preset positions.

In some examples, the detector 20 being controlled to move to a plurality of second preset positions along at least two directions in step 220 may include: controlling the detector 20 to pass one part of the plurality of second preset positions along a first direction and receive X-rays penetrating the stitching body part at the second preset positions passed, and controlling the detector 20 to pass the other part of the plurality of second preset positions along a second direction and receive X-rays penetrating the stitching body part at the second preset positions passed, wherein the first and second directions may form a two-dimensional surface.

In some embodiments, the first and second directions are both straightline directions. In some embodiments, the first and second directions are both non straightline directions. In some embodiments, one of the first and second directions is a straightline direction (such as one of a coronal axis direction, a sagittal axis direction and a vertical axis direction of the object under examination), and the other is a non straightline direction.

In some embodiments, the non straightline directions may include at least one of a folded-line direction, a curve direction and a rotational direction. In some examples, the curve direction may be such as a direction of an arc.

In some examples, in step 220, the detector 20 may be controlled to pass one part of the plurality of second preset positions along a first direction and receive X-rays penetrating the stitching body part at the second preset positions passed, the detector 20 may be controlled to pass another part of the plurality of second preset positions along a second direction and receive X-rays penetrating the stitching body part at the second preset positions passed, and the detector20 may be controlled to pass yet another part (e.g. the remaining part) of the plurality of second preset positions along a third direction and receive X-rays penetrating the stitching body part at the second preset positions passed, wherein the first, second and third directions may form a three-dimensional space.

In some embodiments, the first, second and third directions are all straightline directions. In some embodiments, the first, second and third directions are all non straightline directions. In some embodiments, one of the first, second and third directions is a straightline direction or a non straightline direction.

In some embodiments, the non straightline direction may include at least one of a folded-line direction, a curve direction and a rotational direction. In some examples, the curve direction may be such as a direction of an arc.

In some embodiments, the first direction is one of a coronal axis direction, a sagittal axis direction and a vertical axis direction of the object under examination, and the second direction is another of the coronal axis direction, the sagittal axis direction and the vertical axis direction of the object under examination.

Referring to FIG. 21, a digital X-ray imaging method in some embodiments may include steps as follows:
Step 300: determining a stitching body part of an object under examination;
Step 310: obtaining a plurality of digital X-ray images of the stitching body part. In some examples, the plurality of digital X-ray images may be obtained while the X-ray source 10 and/or the detector 20 are/is controlled to move along at least two directions and the stitching body part is exposed, and the the at least two directions may be capable of determining a two-dimensional surface or a three-dimensional space.

In some embodiments, obtaining a plurality of digital X-ray images of the stitching body part in step 310 may include: obtaining the plurality of digital X-ray images based on historical images.

In some embodiments, obtaining a plurality of digital X-ray images of the stitching body part in step 310 may include: under a current digital imaging mode, obtaining the plurality of digital X-ray images while the X-ray source 10 and/or the detector 20 are/is controlled to move along at least two directions and the stitching body part is exposed.

In some examples, the X-ray source 10 is controlled to move to a plurality of first preset positions along at least two directions and the detector 20 is controlled to remain stationary; and when the X-ray source 10 is moved to the first preset positions, the X-ray source 10 is controlled to emit X-rays to the stitching body part and the detector 20 is controlled to receive X-rays penetrating the stitching body part to obtain the plurality of digital X-ray images. The plurality of digital X-ray images may be used to be stitched to obtain the radiograph of the stitching body part.

In some examples, the detector 20 may be controlled to move to a plurality of second preset positions along at least two directions and the X-ray source 10 is controlled to remain stationary; and when the detector is moved to the second preset positions, the X-ray source 10 is controlled to emit X-rays to the stitching body part and the detector 20 is controlled to receive X-rays penetrating the stitching body part to obtain the plurality of digital X-ray images. The plurality of digital X-ray images may be used to be stitched to obtain the radiograph of the stitching body part.

In some examples, the X-ray source 10 is controlled to move to a plurality of first preset positions along at least two directions and the detector 20 may be controlled to move to second preset positions corresponding to the plurality of first preset positions along at least two directions; and when the X-ray source 10 and the detector 20 are both moved to any of their respective preset positions, the X-ray source 10 is controlled to emit X-rays to the stitching body part and the detector 20 is controlled to receive X-rays penetrating the stitching body part to obtain a plurality of digital X-ray images, and the plurality of digital X-ray images are stitched to obtain a radiograph of the stitching body part.

In some embodiments, the X-ray source being controlled to move to a plurality of first preset positions along at least two directions may include: controlling the X-ray source 10 to pass one part of the plurality of first preset positions along a first direction and emit X-rays to the stitching body part at the first preset positions passed, and controlling the X-ray source 10 to pass the other part of the plurality of first preset positions along a second direction and emit X-rays to the stitching body part at first preset positions passed, wherein the first and second directions form a two-dimensional surface. In some embodiments, the X-ray source 10 may be controlled to move along the first and second directions alternately to pass the plurality of first preset positions. The two-dimensional surface herein may be a planar surface or a curved surface which may be for example a cylindrical surface or an arc surface.

In some embodiments, the first and second directions are both straightline directions. In some embodiments, the first and second directions are both non straightline directions. In some embodiments, one of the first and second directions is a straightline direction (such as one of a coronal axis direction, a sagittal axis direction and a vertical axis direction of the object under examination), and the other is a non straightline direction.

In some embodiments, the non straightline direction may include at least one of a folded-line direction, a curve direction and a rotational direction. In some examples, the curve direction may be such as a direction of an arc.

In some embodiments, the first direction is one of a coronal axis direction, a sagittal axis direction and a vertical axis direction of the object under examination, and the second direction is another of the coronal axis direction, the sagittal axis direction and the vertical axis direction of the object under examination.

In some embodiments, the X-ray source 10 being controlled to move to a plurality of first preset positions along at least two directions may include: controlling the X-ray source 10 to pass one part of the plurality of first preset positions along a first direction and emit X-rays to the stitching body part at the first preset positions passed, controlling the X-ray source 10 to pass another part of the plurality of first preset positions along a second direction and emit X-rays to the stitching body part at the first preset positions passed, and controlling the X-ray source 10 to pass the remaining part of the plurality of first preset positions along a third direction and emit X-rays to the stitching body part at the first preset positions passed, wherein the first, second and third directions form a three-dimensional space.

In some embodiments, the first and second directions are both straightline directions. In some embodiments, the first and second directions are both non straightline directions. In some embodiments, one of the first and second directions is a straightline direction (such as one of a coronal axis direction, a sagittal axis direction and a vertical axis direction of the object under examination), and the other is a non straightline direction.

In some embodiments, the non straightline direction may include at least one of a folded-line direction, a curve direction and a rotational direction. In some examples, the curve direction may be such as a direction of an arc.

In some embodiments, the first direction is one of a coronal axis direction, a sagittal axis direction and a vertical axis direction of the object under examination, and the second direction is another of the coronal axis direction, the sagittal axis direction and the vertical axis direction of the object under examination.

In some embodiments, the detector 20 being controlled to move to a plurality of second preset positions along at least two directions may include: controlling the detector 20 to pass one part of the plurality of second preset positions along a first direction and receive the X-rays penetrating the stitching body part at the second preset positions passed, and controlling the detector 20 to pass the other part of the plurality of second preset positions along a second direction and receive the X-rays penetrating the stitching body part at the second preset positions passed, wherein the first and second directions form a two-dimensional surface. In some embodiments, the detector 20 may be controlled to move along the first and second directions alternately to pass the plurality of second preset positions. The two-dimensional surface herein may be a planar surface or a curved surface which may be for example a cylindrical surface or an arc surface.

In some embodiments, the first and second directions are both straightline directions. In some embodiments, the first and second directions are both non straightline directions. In some embodiments, one of the first and second directions may be a straightline direction (such as one of a coronal axis direction, a sagittal axis direction and a vertical axis direction of the object under examination), and the other may be a non straightline direction.

In some embodiments, the non straightline direction may include at least one of a folded-line direction, a curve direction and a rotational direction. In some examples, the curve direction may be such as a direction of an arc.

In some embodiments, the detector20 being controlled to move to a plurality of second preset positions along at least two directions may include: controlling the detector 20 to pass one part of the plurality of second preset positions along a first direction and receive the X-rays penetrating the stitching body part at the second preset positions passed, controlling the detector 20 to pass another part of the plurality of second preset positions along a second direction and receive the X-rays penetrating the stitching body part at the second preset positions passed, and controlling the detector 20 to pass yet another part (such as the remaining part) of the plurality of second preset positions along a third direction and receive the X-rays penetrating the stitching body part at the second preset positions passed, wherein the first, second and third directions may form a three-dimensional space.

In some embodiments, the first, second and third directions are all straightline directions. In some embodiments, the first, second and third directions are all non straightline directions. In some embodiments, one of the first, second and third directions is a straightline direction or a non straightline direction.

In some embodiments, the non straightline direction may include at least one of a folded-line direction, a curve direction and a rotational direction. In some examples, the curve direction may be such as a direction of an arc.

In some embodiments, the first direction is one of a coronal axis direction, a sagittal axis direction and a vertical axis direction of the object under examination, and the second direction is another of the coronal axis direction, the sagittal axis direction and the vertical axis direction of the object under examination.

Step 320: stitching the plurality of digital X-ray images to obtain a radiograph of the stitching body part.

In some examples, in step 320, at least two digital X-ray images from the plurality of digital X-ray images are stitched in the first stitching direction to obtain an intermediate stitching image, wherein the at least two digital X-ray images are obtained while the X-ray source 10 and/or the detector 20 pass/passes a preset position along the first direction for exposure; and in step 320, the intermediate stitching image and at least one digital X-ray image from the plurality of digital X-ray images are stitched in the second stitching direction to obtain the radiograph of the stitching body part, wherein the at least one digital X-ray image is obtained while the X-ray source 10 and/or the detector 20 pass/passes a preset direction along the second direction for exposure.

In some examples, in step 320, at least two digital X-ray images from the plurality of digital X-ray images are stitched in the second stitching direction to obtain an intermediate stitching image, wherein the at least two digital X-ray images are obtained while the X-ray source 10 and/or the detector 20 pass/passes a preset direction along the second direction for exposure; and in step 320, the intermediate stitching image and the at least one digital X-ray image from the plurality of digital X-ray images are stitched in the first stitching direction to obtain the radiograph of the stitching body part, wherein the at least one digital X-ray image is obtained while the X-ray source 10 and/or the detector 20 pass/passes a preset position along the first direction for exposure.

In some examples, in step 320, at least three digital X-ray images from a plurality of digital X-ray images are stitched in the first stitching direction to obtain at least two intermediate stitching images, wherein the at least three digital X-ray images are obtained while the X-ray source 10 and/or the detector 20 pass/passes a preset position along the first direction for exposure; and in step 320, at least two intermediate stitching images are stitched in the second stitching direction to obtain the radiograph of the stitching body part.

In some examples, in step 320, at least three digital X-ray images from a plurality of digital X-ray images are stitched in the second stitching direction to obtain at least two intermediate stitching images, wherein the at least three digital X-ray images are obtained while the X-ray source 10 and/or the detector 20 pass/passes a preset position along the second direction, and in step 320, at least two intermediate stitching images are stitched in the first stitching direction to obtain the radiograph of the stitching body part.

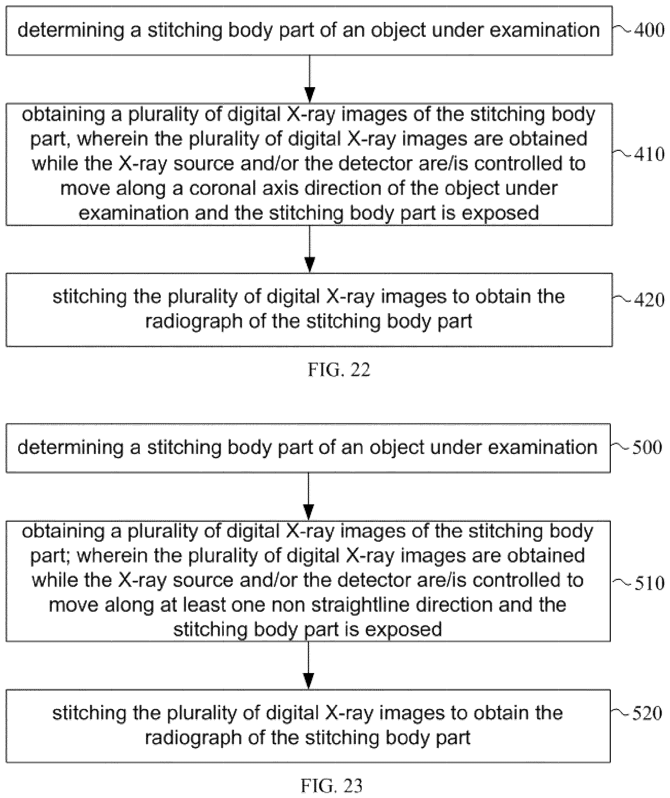

Referring to FIG. 22, a digital X-ray imaging method in some embodiments may include steps as follows:
Step 400: determining a stitching body part of an object under examination.
Step 410: obtaining a plurality of digital X-ray images of the stitching body part, wherein the plurality of digital X-ray images are obtained while the X-ray source 10 and/or the detector 20 are/is controlled to move along a coronal axis direction of the object under examination and the stitching body part is exposed.

In some embodiments, obtaining a plurality of digital X-ray images of the stitching body part in step 410 may include: obtaining the plurality of digital X-ray images based on historical images.

In some embodiments, obtaining a plurality of digital X-ray images of the stitching body part in step 410 may include: under a current digital imaging mode, obtaining the plurality of digital X-ray images while the X-ray source 10 and/or the detector 20 are/is controlled to move along a coronal axis direction of the object under examination and the stitching body part is exposed.

In some embodiments, obtaining the plurality of digital X-ray images while the X-ray source 10 and/or the detector 20 are/is controlled to move along a coronal axis direction of the object under examination and the stitching body part is exposed may include: controlling the X-ray source 10 to move to a plurality of first preset positions along the coronal axis direction of the object under examination and controlling the detector 20 to remain stationary; and when the X-ray source 10 is moved to the first preset positions, obtaining the plurality of digital X-ray images while the X-ray source 10 is controlled to emit X-rays to the stitching body part and the detector 10 is controlled to receive X-rays penetrating the stitching body part.

In some embodiments, obtaining the plurality of digital X-ray images while the X-ray source 10 and/or the detector 20 are/is controlled to move along the coronal axis direction of the object under examination and the stitching body part is exposed may include: controlling the detector 20 to move to a plurality of second preset positions along the coronal axis direction of the object under examination and controlling the X-ray source 10 to remain stationary; and when the detector 20 is moved to the second preset positions, obtaining the plurality of digital X-ray images while the X-ray source 10 is controlled to emit X-rays to the stitching body part and the the detector 20 is controlled to receive X-rays penetrating the stitching body part.

In some embodiments, obtaining the plurality of digital X-ray images while the X-ray source 10 and/or the detector 20 are/is controlled to move along the coronal axis direction of the object under examination and the stitching body part is exposed may include: controlling the X-ray source 10 to move to a plurality of first preset positions along the coronal axis direction of the object under examination and controlling the detector 20 to move to second preset positions corresponding to the plurality of first preset positions along the coronal axis direction of the object under examination; and when the X-ray source 10 and the detector 20 are both moved to any of their respective preset positions, obtaining the plurality of digital X-ray images while the X-ray source 10 is controlled to emit X-rays to the stitching body part and the detector 20 is controlled to receive X-rays penetrating the stitching body part.

Step 420: stitching the plurality of digital X-ray images to obtain the radiograph of the stitching body part.

Referring to FIG. 23, a digital X-ray imaging method in some embodiments may include steps as follows:
Step 500: determining a stitching body part of an object under examination;
Step 510: obtaining a plurality of digital X-ray images of the stitching body part; wherein the plurality of digital X-ray images are obtained while the X-ray source 10 and/or the detector 20 are/is controlled to move along at least one non straightline direction and the stitching body part is exposed.

In some embodiments, obtaining a plurality of digital X-ray images of the stitching body part in step 510 may include: obtaining the plurality of digital X-ray images based on historical images. The historical images here may refer to digital X-ray images that have been exposed and stored in advance.

In some embodiments, obtaining a plurality of digital X-ray images of the stitching body part in step 510 may include: under a current digital imaging mode, obtaining the plurality of digital X-ray images while the X-ray source 10 and/or the detector 20 are/is controlled to move along at least a non straightline direction and the stitching body part is exposed.

In some embodiments, obtaining the plurality of digital X-ray images while the X-ray source 10 and/or the detector 20 are/is controlled to move along at least a non straightline direction and the stitching body part is exposed may include: controlling the X-ray source 10 to move to a plurality of first preset positions along at least a non straightline direction and controlling the detector 20 to remain stationary; and when the X-ray source 10 is moved to the first preset positions, obtaining the plurality of digital X-ray images while X-ray source 10 is controlled to emit X-rays to the stitching body part and the detector 20 is controlled to receive X-rays penetrating the stitching body part.

In some embodiments, obtaining the plurality of digital X-ray images while the X-ray source 10 and/or the detector 20 are/is controlled to move along at least a non straightline direction and the stitching body part is exposed may include: controlling the detector 20 to move to a plurality of second preset positions along at least a non straightline direction and controlling the X-ray source 10 to remain stationary; and when the detector 20 is moved to the second preset positions, obtaining the plurality of digital X-ray images while X-ray source 10 is controlled to emit X-rays to the stitching body part and the detector 20 is controlled to receive the X-rays penetrating the stitching body part.

In some embodiments, obtaining the plurality of digital X-ray images while the X-ray source 10 and/or the detector 20 are/is controlled to move along at least a non straightline direction and the stitching body part is exposed may include: controlling the X-ray source 10 to move to a plurality of first preset positions along at least a non straightline direction and controlling the detector 20 to move to second preset positions corresponding to the plurality of first preset positions along at least a non straightline direction; and when the X-ray source 10 and the detector 20 are both moved to any of their respective preset positions, obtaining the plurality of digital X-ray images while X-ray source 10 is controlled to emit X-rays to the stitching body part and the detector 20 is controlled to receive X-rays penetrating the stitching body part.

In some embodiments, the non straightline direction comprises at least one of a folded-line direction, a curve direction and a rotational direction.

Step 520: stitching the plurality of digital X-ray images to obtain the radiograph of the stitching body part.

It shall be noted that, the movement of the X-ray source 10 herein, such as the displacement of the X-ray source 10, may mainly refer to allowing the X-ray exposure region formed by the X-rays emitted by the X-ray source 10 to displace; and similarly, the turning/rotation of the X-ray source 10 may mainly refer to allowing the X-ray exposure region formed by the X-rays emitted by the X-ray source 10 to turn/rotate.

It shall be noted that, in the embodiments about product and method of the present disclosure, if there are partially identical or similar technical features, they may be referred to each other to avoid duplicate descriptions, such as descriptions about stitching, about the stitching body part, about the movement of the X-ray source and/or the detector, etc..

The present disclosure has been illustrated by reference to various exemplary embodiments. However, those skilled in the art will recognize that the exemplary embodiments can be changed and modified without departing from the scope of the present disclosure. For example, the various operational steps and the components used to perform the operational steps can be implemented in different ways depending on a particular application or taking into account any number of cost functions associated with the operation of the system (for example, one or more steps can be deleted, modified or combined into other steps).

In the embodiments above, this may be accomplished, in whole or in part, by software, hardware, firmware, or any combination thereof. Furthermore, as will be understood by those skilled in the art, the principles of the present disclosure may be embodied in computer program products on computer readable storage media that are preloaded with computer readable program code. Any tangible, non-transitory computer readable storage media may be used, including magnetic storage devices (hard disks, floppy disks, etc.), optical storage devices (CD to ROM, DVD, Blu Ray disks, etc.), flash memory and/or the like. These computer program instructions may be loaded onto a general purpose computer, a special purpose computer, or other programmable data processing device to form a machine so that the instructions executed on the computer or other programmable data processing device generate a device for achieving a specified function. These computer program instructions may also be stored in computer readable memory that may instruct the computer or other programmable data processing device to operate in a particular manner so that the instructions stored in the computer readable memory form a manufactured article including an implementing device for achieving a specified function." A computer program instruction may also be loaded into a computer or other programmable data processing device so as to cause a series of operation steps to be executed on the computer or other programmable device to produce a computer-implemented process, so that the instructions executed on the computer or other programmable device provide the steps for implementing a specified function.

Although the principles of the present disclosure have been shown in various embodiments, many modifications of the structure, arrangement, proportions, elements, materials and parts particularly suited to particular environmental and operational requirements may be used without departing from the principles and scope of this disclosure. The above modifications and other changes or modifications will be included within the scope of this disclosure.

The foregoing specific descriptions have been described with reference to various embodiments. However, those skilled in the art will recognize that various modifications and changes can be made without departing from the scope of this disclosure. Therefore, the consideration of this disclosure will be in an illustrative rather than a restrictive sense and all such modifications will be included within its scope. Also, the advantages, other advantages and solutions to problems with respect to various embodiments have been described above. However, neither the benefits, advantages, solutions to problems nor any elements that generate them or make them more explicit should be construed as critical, necessary or required. The term "include" as used herein, and any other variation thereof, is used non-exclusively so that a process, method, article or device including a list of elements includes not only those elements but also other elements that are not expressly listed or not incorporated into the process, method, system, article or device. Further, the term "couple" as used herein, and any other variation thereof, refers to physical connection, electrical connection, magnetic connection, optical connection, communication connection, functional connection and/or any other connection.

Those skilled in the art will realize that many changes in the details of the above embodiments can be made without departing from the basic principles of the present disclosure. Therefore, the scope of the present disclosure should be determined only by the claims.

## Claims

1. A digital X-ray imaging method, comprising:
displaying options of an exposable body part of an object under examination on a display interface, the options of exposable body part comprising at least an option of a stitching body part;
in response to a selection instruction on the options of the exposable body part, determining a to-be-exposed body part of the object under examination from the options of exposable body part;
when the to-be-exposed body part is a stitching body part, determining an exposure parameter set of the stitching body part, the exposure parameter set comprising multiple travel points that an X-ray source is capable of passing along at least two directions and exposure parameters about the X-ray source at the multiple travel points, and the at least two directions being capable of determining a two-dimensional surface or a three-dimensional space;
in response to an exposure instruction on the stitching body part, controlling, based on the exposure parameter set, the X-ray source to pass the multiple travel points along the at least two directions, and emit X-rays to the stitching body part at each of the travel points passed according to exposure parameters corresponding thereto;
controlling a detector to receive X-rays penetrating the stitching body part to obtain a plurality of digital X-ray images; and
stitching the plurality of digital X-ray images to obtain a radiograph of the stitching body part.

2. The method according to claim 1, wherein determining an exposure parameter set of the stitching body part comprises:
acquiring a predetermined position relationship between the stitching body part and the detector, and determining the exposure parameter set of the stitching body part based on the predetermined position relationship; or,
acquiring a current position relationship between the stitching body part and the detector by a photographic apparatus, and determining the exposure parameter set of the stitching body part based on the current position relationship.

3. The method according to claim 2, wherein determining the exposure parameter set of the stitching body part based on the current position relationship comprises:
displaying on the display interface or projecting by a projection apparatus the current position relationship between the stitching body part and the detector, and determining the exposure parameter set of the stitching body part based on a user operation.

4. The method according to any one of claims 1-3, wherein controlling, based on the exposure parameter set, the X-ray source to pass the multiple travel points along the at least two directions, and emit X-rays to the stitching body part at each of the travel points passed according to exposure parameters corresponding thereto comprises:
based on the exposure parameter set, controlling the X-ray source to pass one part of the multiple travel points along a first direction and emit the X-rays to the stitching body part at each of the travel points passed according to exposure parameters corresponding thereto, and controlling the X-ray source to pass the other part of the multiple travel points along a second direction and emit the X-rays to the stitching body part at each of the travel points passed according to exposure parameters corresponding thereto, the first and second directions forming the two-dimensional surface, wherein the at least two directions comprise the first and second directions, the first and second directions are both straightline directions or non straightline directions, or one of the first and second directions is a straightline direction and the other is a non straightline direction; or,
based on the exposure parameter set, controlling the X-ray source to pass a part of the multiple travel points along a first direction and emit the X-rays to the stitching body part at each of the travel points passed according to exposure parameters corresponding thereto, controlling the X-ray source to pass another part of the multiple travel points along a second direction and emit the X-rays to the stitching body part at each of the travel points passed according to exposure parameters corresponding thereto, and controlling the X-ray source to pass the remaining part of the multiple travel points along a third direction and emit the X-rays to the stitching body part at each of the travel points passed according to exposure parameters corresponding thereto, the first, second and third directions forming the three-dimensional space, wherein the at least two directions comprise the first, second and third directions, the first, second and third directions are all straightline directions or non straightline directions, or one of the first, second and third directions is a straightline direction or a non straightline direction.

5. The method according to claim 4, wherein based on the exposure parameter set, controlling the X-ray source to pass one part of the multiple travel points along a first direction and emit the X-rays to the stitching body part at each of the travel points passed according to exposure parameters corresponding thereto, and controlling the X-ray source to pass the other part of the multiple travel points along a second direction and emit the X-rays to the stitching body part at each of the travel points passed according to exposure parameters corresponding thereto comprise:
based on the exposure parameter set, controlling the X-ray source to move along the first and second directions alternately to pass the multiple travel points.

6. The method according to any one of claims 1-5, wherein controlling a detector to receive X-rays penetrating the stitching body part to obtain a plurality of digital X-ray images comprises:
controlling the detector to move to a position corresponding to each travel point that the X-ray source passes and where the X-ray source emits X-rays and receive X-rays emitted by the X-ray source at said travel point and penetrating the stitching body part to obtain the plurality of digital X-ray images.

7. The method according to claim 4 or 5, wherein the non straightline direction comprises at least one of a folded-line direction, a curve direction and a rotational direction.

8. The method according to claim 4 or 5, wherein the first direction is one of a coronal axis direction, a sagittal axis direction and a vertical axis direction of the object under examination, the second direction is another one of a coronal axis direction, a sagittal axis direction and a vertical axis direction of the object under examination; or at least one of the first and second directions is a rotational direction around a target center corresponding to the X-ray source.

9. The method according to any one of claims 1-5, wherein controlling a detector to receive X-rays penetrating the stitching body part to obtain a plurality of digital X-ray images comprises:
controlling the detector to remain stationary relative to the X-ray source and receive X-rays emitted by the X-ray source at a travel point that the X-ray source passes and penetrating the stitching body part to obtain the plurality of digital X-ray images.

10. The method according to any one of claims 1-9, wherein determining an exposure parameter set of the stitching body part comprises:
acquiring at least one region of interest of the stitching body part and determining the travel point related to the at least one region of interest, the at least one region of interest being located outside a stitching region of the stitching body part.

11. The method according to any one of claims 1-10, wherein the detector and the X-ray source are moved synchronously or successively.

12. A digital X-ray imaging method, comprising:
determining a stitching body part of an object under examination;
determining a digital imaging mode of the stitching body part, the digital imaging mode comprising a first stitching mode and a second stitching mode, wherein the first stitching mode comprises controlling an X-ray source and/or a detector to move along one direction and expose the stitching body part to obtain a plurality of digital X-ray images and stitching the plurality of digital X-ray images to obtain a radiograph of the stitching body part, the second stitching mode comprises controlling an X-ray source and/or a detector to move along at least two directions and expose the stitching body part to obtain a plurality of digital X-ray images, and stitching the plurality of digital X-ray images to obtain a radiograph of the stitching body part, the at least two directions is capable of determining a two-dimensional surface or a three-dimensional space;
when the digital imaging mode is the first stitching mode, controlling the X-ray source and/or the detector to move along one direction and expose the stitching body part to obtain the plurality of digital X-ray images, and stitching the plurality of digital X-ray images to obtain the radiograph of the stitching body part; and
when the digital imaging mode is the second stitching mode, controlling the X-ray source and/or the detector to move along at least two directions and expose the stitching body part to obtain the plurality of digital X-ray images, and stitching the plurality of digital X-ray images to obtain the radiograph of the stitching body part.

13. The method according to claim 12, wherein controlling the X-ray source and/or the detector to move along one direction and expose the stitching body part to obtain the plurality of digital X-ray images, and stitching the plurality of digital X-ray images to obtain the radiograph of the stitching body part comprise:
controlling the X-ray source to move to a plurality of first preset positions along one direction and controlling the detector to remain stationary relative to the X-ray source; when the X-ray source is moved to any of the first preset positions, controlling the X-ray source to emit X-rays to the stitching body part and controlling the detector to receive X-rays penetrating the stitching body part to obtain the plurality of digital X-ray images; and stitching the plurality of digital X-ray images to obtain the radiograph of the stitching body part; or,
controlling the detector to move to a plurality of second preset positions along one direction and controlling the X-ray source to remain stationary relative to the detector; when the detector is moved to any of the second preset positions, controlling the X-ray source to emit X-rays to the stitching body part and controlling the detector to receive X-rays penetrating the stitching body part to obtain the plurality of digital X-ray images; and stitching the plurality of digital X-ray images to obtain the radiograph of the stitching body part; or,
controlling the X-ray source to move to a plurality of first preset positions along one direction and controlling the detector to move along one direction to a plurality of second preset positions corresponding to the first preset positions; when the X-ray source and the detector are both moved to any of their respective preset positions, controlling the X-ray source to emit X-rays to the stitching body part and controlling the detector to receive X-rays penetrating the stitching body part to obtain the plurality of digital X-ray images; and stitching the plurality of digital X-ray images to obtain a radiograph of the stitching body part.

14. The method according to claim 12, wherein controlling the X-ray source and/or the detector to move along at least two directions and expose the stitching body part to obtain the plurality of digital X-ray images, and stitching the plurality of digital X-ray images to obtain the radiograph of the stitching body part comprise:
controlling the X-ray source to move to a plurality of first preset positions along at least two directions and controlling the detector to remain stationary relative to the X-ray source; when the X-ray source is moved to any of the first preset positions, controlling the X-ray source to emit X-rays to the stitching body part and controlling the detector to receive X-rays penetrating the stitching body part to obtain the plurality of digital X-ray images; and stitching the plurality of digital X-ray images to obtain a radiograph of the stitching body part; or,
controlling the detector to move along at least two directions to a plurality of second preset positions and controlling the X-ray source to remain stationary relative to the detector; when the detector is moved to any of the second preset positions, controlling the X-ray source to emit X-rays to the stitching body part and controlling the detector to receive X-rays penetrating the stitching body part to obtain the plurality of digital X-ray images; and stitching the plurality of digital X-ray images to obtain a radiograph of the stitching body part; or,
controlling the X-ray source to move to a plurality of first preset positions along at least two directions and controlling the detector to move along at least two directions to a plurality of second preset positions corresponding to the first preset positions; when the X-ray source and the detector are both moved to any of their respective preset positions, controlling the X-ray source to emit X-rays to the stitching body part and controlling the detector to receive X-rays penetrating the stitching body part to obtain the plurality of digital X-ray images; and stitching the plurality of digital X-ray images to obtain a radiograph of the stitching body part.

15. The method according to claim 14, wherein controlling the X-ray source to move to a plurality of first preset positions along at least two directions comprises:
controlling the X-ray source to pass one part of the plurality of first preset positions along a first direction and emit X-rays to the stitching body part at any of the first preset positions passed, and controlling the X-ray source to pass the other part of the plurality of first preset positions along a second direction and emit X-rays to the stitching body part at any of the first preset positions passed, the first and second directions forming the two-dimensional surface, wherein the at least two directions comprise the first and second directions, the first and second directions are both straightline directions or non straightline directions, or one of the first and second directions is a straightline direction and the other is a non straightline direction; or,
controlling the X-ray source to pass a part of the plurality of first preset positions along a first direction and emit X-rays to the stitching body part at any of the first preset positions passed, controlling the X-ray source to pass another part of plurality of first preset positions along a second direction and emit X-rays to the stitching body part at any of the first preset positions passed, and controlling the X-ray source to pass the remaining part of plurality of first preset positions along a third direction and emit X-rays to the stitching body part at any of the first preset positions passed, the first, second and third directions forming the three-dimensional space, wherein the at least two directions comprise the first, second and third directions, the first, second and third directions are all straightline directions or non straightline directions, or one of the first, second and third directions is a straightline direction or a non straightline direction.

16. The method according to claim 15, wherein controlling the X-ray source to pass one part of the plurality of first preset positions along a first direction and emit X-rays to the stitching body part at any of the first preset positions passed, and controlling the X-ray source to pass the other part of the plurality of first preset positions along a second direction and emit X-rays to the stitching body part at any of the first preset positions passed comprise:
controlling the X-ray source to move along the first and second directions alternately to pass the plurality of first preset positions.

17. The method according to claim 14, wherein controlling the detector to move along at least two directions to a plurality of second preset positions comprises:
controlling the detector to pass one part of the plurality of second preset positions along a first direction and receive X-rays penetrating the stitching body part at any of the second preset positions passed, and controlling the detector to pass the other part of the plurality of second preset positions along a second direction and receive X-rays penetrating the stitching body part at any of the second preset positions passed, the first and second directions forming the two-dimensional surface, wherein the at least two directions comprise the first and second directions, the first and second directions are both straightline directions or non straightline directions, or one of the first and second directions is a straightline direction or a non straightline direction; or,
controlling the detector to pass a part of the plurality of second preset positions along a first direction and receive X-rays penetrating the stitching body part at any of the second preset positions passed, controlling the detector to pass another part of the plurality of second preset positions along a second direction and receive X-rays penetrating the stitching body part at any of the second preset positions passed, and controlling the detector to pass the remain part of the plurality of second preset positions along a third direction and receive X-rays penetrating the stitching body part at any of the second preset positions passed, the first, second and third directions forming the three-dimensional space, wherein the at least two directions comprise the first, second and third directions, the first, second and third directions are all straightline directions or non straightline directions, or one of the first, second and third directions is a straightline direction or a non straightline direction.

18. The method according to any of claims 15-17, wherein the first direction is one of a coronal axis direction, a sagittal axis direction and a vertical axis direction of the object under examination, and the second direction is another one of a coronal axis direction, a sagittal axis direction and a vertical axis direction of the object under examination; or at least one of the first and second directions is a rotational direction around a target center corresponding to the X-ray source.

19. The method according to any one of claims 15-17, wherein the non straightline direction comprises at least one of a folded-line direction, a curve direction and a rotational direction.

20. The method according to any one of claims 12-19, wherein determining a digital imaging mode of the stitching body part comprises:
displaying options of digital imaging mode on a display interface, and determining the digital imaging mode in response to a selection instruction on the options of digital imaging mode; or,
automatically determining the digital imaging mode according to the stitching body part.

21. The method according to any one of claims 12-20, further comprising:
switching among digital imaging modes in response to a triggering of a mode switching button.

22. A digital X-ray imaging method, comprising:
determining a stitching body part of an object under examination;
obtaining a plurality of digital X-ray images of the stitching body part, wherein the plurality of digital X-ray images are obtained while an X-ray source and/or a detector are/is controlled to move along at least two directions and the stitching body part is exposed, the at least two directions are capable of determining a two-dimensional surface or a three-dimensional space; and
stitching the plurality of digital X-ray images to obtain a radiograph of the stitching body part.

23. The method according to claim 22, wherein obtaining a plurality of digital X-ray images of the stitching body part comprises:
obtaining the plurality of digital X-ray images based on historical images; or,
under a current digital imaging mode, obtaining the plurality of digital X-ray images while the X-ray source and/or the detector are/is controlled to move along at least two directions and the stitching body part is exposed.

24. The method according to claim 23, wherein obtaining the plurality of digital X-ray images while the X-ray source and/or the detector are/is controlled to move along at least two directions and the stitching body part is exposed comprises:
controlling the X-ray source to move to a plurality of first preset positions along at least two directions and controlling the detector to remain stationary relative to the X-ray source, and when the X-ray source is moved to any of the first preset positions, obtaining the plurality of digital X-ray images while the X-ray source is controlled to emit X-rays to the stitching body part and the detector is controlled to receive X-rays penetrating the stitching body part; or,
controlling the detector to move to a plurality of second preset positions along at least two directions and controlling the X-ray source to remain stationary relative to the detector, when the detector is moved to any of the second preset positions, obtaining the plurality of digital X-ray images while the X-ray source is controlled to emit X-rays to the stitching body part and the detector is controlled to receive X-rays penetrating the stitching body part; or,
controlling the X-ray source to move to a plurality of first preset positions along at least two directions and controlling the detector to move to second preset positions corresponding to the plurality of first preset positions along at least two directions; when the X-ray source and the detector are both moved to any of their respective preset positions, obtaining the plurality of digital X-ray images while the X-ray source is controlled to emit X-rays to the stitching body part and the detector is controlled to receive X-rays penetrating the stitching body part; and stitching the plurality of digital X-ray images to obtain a radiograph of the stitching body part.

25. The method according to claim 24, wherein controlling the X-ray source to move to a plurality of first preset positions along at least two directions comprises:
controlling the X-ray source to pass one part of the plurality of first preset positions along a first direction and emit X-rays to the stitching body part at any of the first preset positions passed, and controlling the X-ray source to pass the other part of the plurality of first preset positions along a second direction and emit X-rays to the stitching body part at any of the first preset positions passed, the first and second directions forming the two-dimensional surface, wherein the at least two directions comprises the first and second directions, the first and second directions are both straightline directions or non straightline directions, or one of the first and second directions is a straightline direction or a non straightline direction; or,
controlling the X-ray source to pass a part of the plurality of first preset positions along a first direction and emit X-rays to the stitching body part at any of the first preset positions passed, controlling the X-ray source to pass another part of the plurality of first preset positions along a second direction and emit X-rays to the stitching body part at any of the first preset positions passed, and controlling the X-ray source to pass the remain part of the plurality of first preset positions along a third direction and emit X-rays to the stitching body part at any of the first preset positions passed, the first, second and third directions forming three-dimensional space, wherein the at least two directions comprise the first, second and third directions, the first, second and third directions are all straightline directions or non straightline directions, or one of the first, second and third directions is a straightline direction or a non straightline direction.

26. The method according to claim 25, wherein controlling the X-ray source to pass one part of the plurality of first preset positions along a first direction and emit X-rays to the stitching body part at any of the first preset positions passed, and controlling the X-ray source to pass the other part of the plurality of first preset positions along a second direction and emit X-rays to the stitching body part at any of the first preset positions passed comprise:
controlling the X-ray source to move along the first and second directions alternately to pass the plurality of first preset positions.

27. The method according to claim 24, wherein controlling the detector to move to a plurality of second preset positions along at least two directions comprises:
controlling the detector to pass one part of the plurality of second preset positions along a first direction and receive X-rays penetrating the stitching body part at any of the second preset positions passed, and controlling the detector to pass the other part of the plurality of second preset positions along a second direction and receive X-rays penetrating the stitching body part at any of the second preset positions passed, the first and second directions forming the two-dimensional surface, wherein the at least two directions comprise the first and second directions, the first and second directions are both straightline directions or non straightline directions, or one of the first and second directions is a straightline direction or a non straightline direction; or,
controlling the detector to pass a part of the plurality of second preset positions along a first direction and receive X-rays penetrating the stitching body part at any of the second preset positions passed, controlling the detector to pass another part of the plurality of second preset positions along a second direction and receive X-rays penetrating the stitching body part at any of the second preset positions passed, and controlling the detector to pass the remain part of the plurality of second preset positions along a third direction and receive X-rays penetrating the stitching body part at any of the second preset positions passed, the first, second and third directions forming the three-dimensional space, wherein the at least two directions comprise the first, second and third directions, the first, second and third directions are all straightline directions or non straightline directions, or one of the first, second and third directions is a straightline direction or a non straightline direction.

28. The method according to any of claims 25-27, wherein the first direction is one of a coronal axis direction, a sagittal axis direction and a vertical axis direction of the object under examination, and the second direction is another one of a coronal axis direction, a sagittal axis direction and a vertical axis direction of the object under examination; or at least one of the first and second directions is a rotational direction around a target center corresponding to the X-ray source.

29. The method according to any one of claims 25-27, wherein the non straightline direction comprises at least one of a folded-line direction, a curve direction and a rotational direction.

30. The method according to any one of claims 25-27, wherein stitching the plurality of digital X-ray images to obtain a radiograph of the stitching body part comprises:
stitching the plurality of digital X-ray images in at least two stitching directions to obtain a radiograph of the stitching body part.

31. The method according to claim 30, wherein stitching the plurality of digital X-ray images in at least two stitching directions to obtain a radiograph of the stitching body part comprises:
stitching at least two digital X-ray images from the plurality of digital X-ray images in a first stitching direction to obtain an intermediate stitching image, said at least two digital X-ray images being obtained while the X-ray source and/or the detector pass/passes a preset position along the first direction for exposure; and stitching the intermediate stitching image and at least one digital X-ray image from the plurality of digital X-ray images in a second stitching direction to obtain the radiograph of the stitching body part, said at least one digital X-ray image being obtained while the X-ray source and/or the detector pass/passes a preset position along the second direction for exposure; or,
stitching at least two digital X-ray images from the plurality of digital X-ray images in a second stitching direction to obtain an intermediate stitching image, said at least two digital X-ray images being obtained while the X-ray source and/or the detector pass/passes at a preset position along the second direction for exposure; and stitching the intermediate stitching image and at least one digital X-ray image from the plurality of digital X-ray images in a first stitching direction to obtain a radiograph of the stitching body part, said at least one digital X-ray image being obtained while the X-ray source and/or the detector pass/passes at a preset position along the first direction for exposure; or,
stitching at least three digital X-ray images from the plurality of digital X-ray images in a first stitching direction to obtain at least two intermediate stitching images, said at least three digital X-ray images being obtained while the X-ray source and/or the detector pass/passes at a preset position along the first direction for exposure; and stitching the at least two intermediate stitching images in a second stitching direction to obtain a radiograph of the stitching body part; or,
stitching at least three digital X-ray images from the plurality of digital X-ray images in a second stitching direction to obtain at least two intermediate stitching images, said at least three digital X-ray images being obtained while the X-ray source and/or the detector pass/passes at a preset position along the second direction for exposure; and stitching the at least two intermediate stitching images in a first stitching direction to obtain a radiograph of the stitching body part.

32. A digital X-ray imaging method, comprising:
determining a stitching body part of an object under examination;
obtaining a plurality of digital X-ray images of the stitching body part, wherein the plurality of digital X-ray images are obtained while an X-ray source and/or a detector are/is controlled to move along a coronal axis direction of the object under examination and the stitching body part is exposed; and
stitching the plurality of digital X-ray images to obtain a radiograph of the stitching body part.

33. The method according to claim 32, wherein obtaining a plurality of digital X-ray images of the stitching body part comprises:
obtaining the plurality of digital X-ray images based on historical images; or,
under a current digital imaging mode, obtaining the plurality of digital X-ray images while the X-ray source and/or the detector are/is controlled to move along the coronal axis direction of the object under examination and the stitching body part is exposed.

34. The method according to claim 33, wherein obtaining the plurality of digital X-ray images while the X-ray source and/or the detector are/is controlled to move along the coronal axis direction of the object under examination and the stitching body part is exposed comprises:
controlling the X-ray source to move to a plurality of first preset positions along the coronal axis direction of the object under examination and controlling the detector to remain stationary relative to the X-ray source, and when the X-ray source is moved to any of the first preset positions, obtaining the plurality of digital X-ray images while the X-ray source is controlled to emit X-rays to the stitching body part and the detector is controlled to receive X-rays penetrating the stitching body part; or,
controlling the detector to move to a plurality of second preset positions along the coronal axis direction of the object under examination and controlling the X-ray source to remain stationary relative to the detector, and when the detector is moved to any of the second preset positions, obtaining the plurality of digital X-ray images while the X-ray source is controlled to emit X-rays to the stitching body part and the detector is controlled to receive X-rays penetrating the stitching body part; or,
controlling the X-ray source to move to a plurality of first preset positions along the coronal axis direction of the object under examination and controlling the detector to move to second preset positions corresponding to the plurality of first preset positions along the coronal axis direction of the object under examination, and when the X-ray source and the detector are both moved to any of their respective preset positions, obtaining the plurality of digital X-ray images while the X-ray source is controlled to emit X-rays to the stitching body part and the detector is controlled to receive X-rays penetrating the stitching body part.

35. A digital X-ray imaging method, comprising:
determining a stitching body part of an object under examination;
obtaining a plurality of digital X-ray images of the stitching body part, wherein the plurality of digital X-ray images are obtained while an X-ray source and/or a detector are/is controlled to move along at least one non straightline direction and the stitching body part is exposed; and
stitching the plurality of digital X-ray images to obtain a radiograph of the stitching body part.

36. The method according to claim 35, wherein obtaining a plurality of digital X-ray images of the stitching body part comprises:
obtaining the plurality of digital X-ray images based on historical images; or,
under a current digital imaging mode, obtaining the plurality of digital X-ray images while the X-ray source and/or the detector are/is controlled to move along at least one non straightline direction and the stitching body part is exposed.

37. The method according to claim 36, wherein obtaining the plurality of digital X-ray images while the X-ray source and/or the detector are/is controlled to move along at least one non straightline direction and the stitching body part is exposed comprises:
controlling the X-ray source to move to a plurality of first preset positions along at least one non straightline direction and controlling the detector to remain stationary relative to the X-ray source, and when the X-ray source is moved to any of the first preset positions, obtaining the plurality of digital X-ray images while the X-ray source is controlled to emit X-rays to the stitching body part and the detector is controlled to receive X-rays penetrating the stitching body part; or,
controlling the detector to move to a plurality of second preset positions along at least one non straightline direction and controlling the X-ray source to remain stationary relative to the detector, and when the detector is moved to any of the second preset positions, obtaining the plurality of digital X-ray images while the X-ray source is controlled to emit X-rays to the stitching body part and the detector is controlled to receive X-rays penetrating the stitching body part; or,
controlling the X-ray source to move to a plurality of first preset positions along at least one non straightline direction and controlling the detector to move to second preset positions corresponding to the plurality of first preset positions along at least one non straightline direction, and when the X-ray source and the detector are both moved to any of their respective preset positions, obtaining the plurality of digital X-ray images while the X-ray source is controlled to emit X-rays to the stitching body part and the detector is controlled to receive X-rays penetrating the stitching body part.

38. The method according to any one of claims 35-37, wherein the non straightline direction comprises at least one of a folded-line direction, a curve direction and a rotational direction.

39. A digital X-ray imaging apparatus, comprising:
an X-ray source configured to emit X-rays to an object under examination;
a detector configured to receive the X-rays penetrating the object under examination; and
a processor configured to execute the method according to any one of claims 1 to 38.
